# EUROPEAN PATENT SPECIFICATION

(11) **EP 3 161 008 B1**
(45) Date of publication and mention of the grant of the patent: **25.09.2019**
(21) Application number: 15733415.2
(22) Date of filing: 25.06.2015
(51) Int. Cl.: C07K 16/46

(54) **MULTI-SPECIFIC ANTIBODY CONSTRUCTS**
MULTISPEZIFISCHE ANTIKÖRPERKONSTRUKTE
CONSTRUCTIONS D'ANTICORPS MULTI-SPÉCIFIQUES

(30) Priority: 26.06.2014 GB 201411420
(43) Date of publication of application: 03.05.2017
(73) Proprietor: UCB Biopharma SPRL, 1070 Brussels (BE)
(72) Inventor: BHATTA, Pallavi, Slough Berkshire SL1 3WE (GB); DAVE, Emma, Slough Berkshire SL1 3WE (GB); HEYWOOD, Sam Philip, Slough Berkshire SL1 3WE (GB); HUMPHREYS, David Paul, Slough Berkshire SL1 3WE (GB)
(74) Representative: UCB Intellectual Property
(86) International application number: PCT/EP2015/064450
(87) International publication number: WO 2015/197789

(56) References cited:
- EP-A1- 2 535 349
- WO-A1-2012/025530
- US-A1- 2010 081 796
- REBECCA CROASDALE ET AL: "Development of tetravalent IgG1 dual targeting IGF-1R-EGFR antibodies with potent tumor inhibition", ARCHIVES OF BIOCHEMISTRY AND BIOPHYSICS, vol. 526, no. 2, 1 October 2012 (2012-10-01), pages 206-218, XP055044587, ISSN: 0003-9861, DOI: 10.1016/j.abb.2012.03.016
- SCHANZER J ET AL: "Development of Tetravalent, Bispecific CCR5 Antibodies with Antiviral Activity against CCR5 Monoclonal Antibody-Resistant HIV-1 Strains", ANTIMICROBIAL AGENTS AND CHEMOTHERAPY, AMERICAN SOCIETY FOR MICROBIOLOGY, vol. 55, no. 5, 1 May 2011 (2011-05-01), pages 2369-2378, XP002700795, ISSN: 0066-4804, DOI: 10.1128/AAC.00215-10 [retrieved on 2011-02-07]
- S. METZ ET AL: "Bispecific antibody derivatives with restricted binding functionalities that are activated by proteolytic processing", PROTEIN ENGINEERING DESIGN AND SELECTION, vol. 25, no. 10, 1 October 2012 (2012-10-01), pages 571-580, XP55069824, ISSN: 1741-0126, DOI: 10.1093/protein/gzs064

## Description

The present disclosure relates to certain multi-specific antibody constructs, pharmaceutical formulations comprising the construct, DNA encoding the constructs and vectors comprising same. The disclosure also extends to a method of expressing the constructs, for example in a host cell and methods for formulating same as a pharmaceutical composition. The disclosure also relates to use of the multi-specific antibody constructs and formulations in treatment.

There are a number of approaches for generating bi-specific antibodies, many of which are based on a format originally described by Morrison *et al* (Coloma and Morrison 1997, Nat Biotechnol. 15, 159-163) involving the fusion of single chain variable fragments (scFv) to whole antibodies, e.g. IgG, or to antibody fragments, e.g. Fab or F(ab')₂.

US2010/081796 describes bispecific antibodies having the Ig-dsscFv2 format. WO2012/025530 describes bispecific antibodies having the Ig-dsFv format comprising only one dsFv fragment linked to the C-terminal of the heavy chain of the full-length Ig.

US2008/0050370 discloses certain bi-specific antibody molecules which comprise 'stabilised' scFvs. The molecules of this type have good binding affinity for the antigens to which they are specific and no significant occlusion of antigen binding sites occurs in the format. The V_{H} and V_{L} domains of the scFvs used in such bi-specific molecules are held together using peptide linkers. However, for certain combinations of V_{H} and V_{L} variable domains, the peptide linkers are unable to confer sufficient stability to the scFv, resulting in variable domain 'breathing' and promiscuous intermolecular pairing with variable domains and thus a tendency for molecules to form multimers during expression and post purification through the single chain Fv portion thereof.

The present inventors have re-engineered the multi-specific antibody molecules concerned to provide antibody molecules with equivalent functionality, whilst minimising aggregation during expression and post purification, which facilitates increasing the yield of "monomeric" material obtained and provides molecules after purification, concentration and formulation of suitable stability for use in treatment.

Thus in one aspect, the present disclosure provides a multi-specific antibody molecule comprising or consisting of:
a) a polypeptide chain of formula (**I**):

   **V_{H}-CH₁-CH₂-CH₃-X-(V₁)ₚ**;

   and
b) a polypeptide chain of formula (**II**):

   **V_{L}-C_{L}-Y-(V₂)_{q};**

   wherein:
   V_{H} represents a heavy chain variable domain;
   CH₁ represents a domain of a heavy chain constant region, for example domain 1 thereof;
   CH₂ represents a domain of a heavy chain constant region, for example domain 2 thereof;
   CH₃ represents a domain of a heavy chain constant region, for example domain 3 thereof;
   X represents a bond or linker;
   Y represents a bond or linker;
   V₁ represents a dsFv;
   V_{L} represents a variable domain, for example a light chain variable domain;
   C_{L} represents a domain from a constant region, for example a light chain constant region domain, such as Ckappa;
   V₂ represents a dsFv;
   p represents 0 or 1;
   q represents 0 or 1;
   wherein at least one of p or q is 1, and
   when p is 1, q is 0, and Y is absent, and
   when q is 1, p is 0, and X is absent, and
   when q is 1 and V₂ is a dsFv, the VL domain of V₂ is attached to Y, and the molecule is provided as dimer with two heavy chains of formula (I) and two associated light chains of formula (**II**).

In one embodiment p is 1, V₁ is dsFv and q is 0 and there are at least two polypeptide chains of formula (**I**), for example as a full length antibody format with two heavy chains (of formula (**I**)) and two light chains (of formula (**II**)) assembled in a Y shaped molecule.

In one embodiment, there is provided a multi-specific antibody molecule, comprising or consisting of:
a) a polypeptide chain of formula (**Ia**):
   **V_{H}-CH₁-CH₂-CH₃-X-V₁;**
b) a polypeptide chain of formula (**IIa**):

   **V_{L}-C_{L};**

   wherein:
   V_{H}, CH₁, CH₂, CH₃, X, V_{L}, C_{L} and V₁ are as defined above.

When V₁ in the polypeptide of formula (**Ia**) is a dsFv then the multi-specific antibody will comprise a third polypeptide encoding the corresponding free V_{H} or V_{L} domain which is not attached to X. This "free variable domain" will generally be common in both heavy chains and bind to the variable domain attached to X by virtue of the disulphide bond. Thus whilst the actual variable domain fused or linked via X to the polypeptide may be different in the two heavy chains in the form a "full length antibody" the free variable domains paired therewith will generally be identical to each other. Typically the variable domain fused or linked via X to the rest of the polypeptide will be identical in both heavy chains of the dimer.

In one embodiment there is provided a dimer comprising two polypeptides of formula (**I**) wherein V₁ in each polypeptide is identical, and optionally further comprising two polypeptides of formula (**II**).

In one embodiment, there is provided a multi-specific antibody molecule, comprising or consisting of:
a) a polypeptide chain of formula (**Ib**):

   **V_{H}-CH₁-CH₂-CH₃**;

   and
b) a polypeptide chain of formula (**II**b):

   **V_{L}-C_{L}-Y-V₂;**

   wherein:
   V_{H}, CH₁, CH₂, CH₃, Y, V_{L}, C_{L}, Y and V₂ are as defined above.

When V₂ in the polypeptide of formula (**IIb**) is a dsFv then the multi-specific antibody will comprise a third polypeptide encoding the corresponding free V_{H} or V_{L} domain which is not attached to Y. This "free variable domain" will be common in both light chains and bind to the variable domain attached to Y by virtue of the disulphide bond. Thus whilst the actual variable domain fused or linked via Y to the polypeptide may be different in the two light chains in the form a "full length antibody" the free variable domains paired therewith will generally be identical to each other. Typically the variable domain fused or linked via X to the rest of the polypeptide will be identical in both light chains of the dimer.

In one embodiment where p is 0, X is absent. In one embodiment where q is 0, Y is absent.

Advantageously, the multi-specific antibody molecules of the present disclosure minimises the amount of aggregation seen after purification and maximise the amount of monomer in the formulations of the construct at pharmaceutical concentrations, for example the monomer may be 50%, 60%, 70% or 75% or more, such as 80, 90, 91, 92, 93, 94, 95, 96, 97, 98 or 99% or more of the protein purified is present as "monomer".

### Detailed Description of the Invention

"Multi-specific antibody" as employed herein refers to an antibody molecule as described herein which has two or more binding domains, for example two or three binding domains.

In one embodiment the antibody construct is a tri-specific antibody.

"Tri-specific antibody" as employed herein refers to an antibody molecule with three antigen binding sites, which may independently bind the same or different antigens.

In one embodiment the construct is a bi-specific antibody.

"Bi-specific molecule" as employed herein refers to a molecule with two antigen binding sites, which may bind the same or different antigens.

In one embodiment the domains all bind the same antigen, including binding the same epitope on the antigen or binding different epitopes on the antigen.

In one embodiment there are three binding domains and each of the three binding domains bind different (distinct) antigens.

In one embodiment there are three binding domains and two binding domains bind the same antigen, including binding the same epitope or different epitopes on the same antigen, and the third binding domain binds a different (distinct) antigen.

In one embodiment the multi-specific antibody molecule of the present disclosure has only two antigen binding sites. It will be appreciated that where the multi-specific antibody molecule dimerises such that there are four antigen binding sites these will typically still only bind to two different antigens. Thus in one embodiment there are four binding domains of which one pair of binding domains bind one antigen and the other pair of binding domains bind a second different antigen.

"Antigen binding site" as employed herein refers to a portion of the molecule, which comprises a pair of variable regions, in particular a cognate pair, that interact specifically with the target antigen.

Binding domains as employed herein includes a single domain antibody i.e. a variable region and antigen binding sites.

"Specifically" as employed herein is intended to refer to a binding site that only recognises the antigen to which it is specific or a binding site that has significantly higher binding affinity to the antigen to which is specific compared to affinity to antigens to which it is non-specific, for example 5, 6, 7, 8, 9, 10 times higher binding affinity.

Binding affinity may be measured by standard assay, for example surface plasmon resonance, such as BIAcore.

"Protein" as employed herein refers to an amino acid sequence of 100 amino acids or more. In one embodiment a "protein" as employed herein refers to an amino acid sequence with a secondary or tertiary structure. In one embodiment a protein is not so much the number of amino acids comprising the basic sequence but is dictated by the level of secondary and/or tertiary structure, folding, in the molecule.

The term "antibody" as used herein refers to an immunoglobulin molecule capable of specific binding to a target antigen, such as a carbohydrate, polynucleotide, lipid, polypeptide, peptide etc., via at least one antigen recognition site (also referred to as a binding site herein), located in the variable region of the immunoglobulin molecule.

As used herein "antibody molecule" includes antibodies and binding fragments thereof.

"Antibody fragments" as employed herein refer to antibody binding fragments including but not limited to Fab, modified Fab, Fab', modified Fab', F(ab')2, Fv, single domain antibodies, scFv, bi, tri or tetra-valent antibodies, Bis-scFv, diabodies, triabodies, tetrabodies and epitope-binding fragments of any of the above (see for example Holliger and Hudson, 2005, Nature Biotech. 23(9):1126-1136; Adair and Lawson, 2005, Drug Design Reviews - Online 2(3), 209-217). The methods for creating and manufacturing these antibody fragments are well known in the art (see for example Verma et al 1998, Journal of Immunological Methods, 216:165-181). Other antibody fragments for use in the present disclosure include the Fab and Fab' fragments described in International patent applications WO05/003169, WO05/003170 and WO05/003171. Multi-valent antibodies may comprise multiple specificities e.g. bispecific or may be monospecific (see for example WO92/22853, WO05/113605, WO2009/040562 and WO2010/035012).

A "binding fragment" as employed herein refers to a fragment capable of binding a target peptide or antigen with sufficient affinity to characterise the fragment as specific for the peptide or antigen.

The term "Fab fragment" as used herein refers to an antibody fragment comprising a light chain fragment comprising a VL (variable light) domain and a constant domain of a light chain (CL), and a VH (variable heavy) domain and a first constant domain (CH1) of a heavy chain. A Fab fragment does not generally comprise a hinge region, whereas a Fab' comprising a hinge region.

The constant region domains of an antibody molecule of the present disclosure may be selected having regard to the proposed function of the antibody molecule, and in particular the effector functions which may be required. For example, the constant region domains may be human IgA, IgD, IgE, IgG or IgM domains. In particular, human IgG constant region domains may be used, especially of the IgG1 and IgG3 isotypes when the antibody molecule is intended for therapeutic uses and antibody effector functions are required. Alternatively, IgG2 and IgG4 isotypes may be used when the antibody molecule is intended for therapeutic purposes and antibody effector functions are not required.

In one embodiment constant domains employed in constructs of the present disclosure are an IgG isotype, for example independently selected from IgG1, IgG2, IgG3, IgG4 and combinations thereof, in particular the isotype IgG1 or IgG4.

It will be appreciated that sequence variants of these constant region domains may also be used. For example IgG4 molecules in which the serine at position 241 has been changed to proline as described in Angal et al 1993, Molecular Immunology, 1993, 30:105-108 may be used. Accordingly, in the embodiment where the antibody is an IgG4 antibody, the antibody may include the mutation S241P.

It will also be understood by one skilled in the art that antibodies may undergo a variety of posttranslational modifications. The type and extent of these modifications often depends on the host cell line used to express the antibody as well as the culture conditions. Such modifications may include variations in glycosylation, methionine oxidation, diketopiperazine formation, aspartate isomerization and asparagine deamidation. A frequent modification is the loss of a carboxy-terminal basic residue (such as lysine or arginine) due to the action of carboxypeptidases (as described in Harris, RJ. Journal of Chromatography 705:129-134, 1995). Accordingly, the C-terminal lysine of the antibody heavy chain may be absent.

When V₁ is a dsFv in compounds of the present disclosure it will be apparent to a person skilled in the art that the polypeptide of formula (**I**) (or another formula as described herein) includes a single variable domain (a component of V₁) linked via a disulphide bond to a variable domain which is part of a fused polypeptide of formula (I).

When V₂ is a dsFv in compounds of the present disclosure it will be apparent to a person skilled in the art that the polypeptide of formula (**I**) (or another formula as described herein) includes a single variable domain (a component of V₂) linked via a disulphide bond to a variable domain which is part of a fused polypeptide of formula (**I**).

Fusion polypeptide as employed herein is a continuous polypeptide chain, generally comprising components not naturally found together, for example prepared by recombinant techniques.

In one embodiment, the multi-specific antibody molecule according to the present disclosure is provided as a dimer of a heavy and light chain of:
Formula (**I**) and (**II**) respectively, wherein the V_{H}-CH₁ portion together with the V_{L}-C_{L} portion form a functional Fab or Fab' fragment; or
Formula (**Ia**) and (**IIa**) respectively, wherein the V_{H}-CH₁ portion together with the V_{L}-C_{L} portion form a functional Fab or Fab' fragment; or
Formula **(Ib)** and **(IIb)** respectively, wherein the V_{H}-CH₁ portion together with the V_{L}-C_{L} portion form a functional Fab or Fab' fragment.

In another embodiment, the multi-specific antibody molecule according to the present disclosure is provided as a tetramer comprising 2X dimers of a heavy and light chain of:
Formula **(I)** and **(II)** respectively, wherein the V_{H}-CH₁- CH₂- CH₃ portion together with the V_{L}-C_{L} portion, for example form a functional IgG; or
Formula **(Ia)** and **(IIa)** respectively, wherein the V_{H}-CH₁- CH₂- CH₃ portion together with the V_{L}-C_{L} portion, for example form a functional IgG; or
Formula **(Ib)** and **(IIb)** respectively, wherein the V_{H}-CH₁- CH₂- CH₃ portion together with the V_{L}-C_{L} portion form a functional IgG.

V_{H} represents a variable domain, for example a heavy chain variable domain. In one embodiment V_{H} represents a heavy chain variable domain. In one embodiment V_{H} is a chimeric variable domain, that is to say it comprises components derived from at least two species, for example a human framework and non-human CDRs. In one embodiment V_{H} is humanised. In one embodiment the V_{H} is human.

V_{L} represents a variable domain, for example a light chain variable domain. In one embodiment V_{L} represents a light chain variable domain. In one embodiment V_{L} is a chimeric variable domain, that is to say it comprises components derived from at least two species, for example a human framework and non-human CDRs. In one embodiment V_{L} is humanised. In one embodiment the V_{L} is human.

Generally V_{H} and V_{L} together form an antigen binding domain. In one embodiment V_{H} and V_{L} form a cognate pair.

"Cognate pair" as employed herein refers to a pair of variable domains from a single antibody, which was generated *in vivo,* i.e. the naturally occurring pairing of the variable domains isolated from a host. A cognate pair is therefore a VH and VL pair. In one example the cognate pair bind the antigen co-operatively. Cognate pairs may be advantageous because they are often affinity matured in the host and therefore may have high affinity for the antigen to which they are specific.

"Variable region" as employed herein refers to the region in an antibody chain comprising the CDRs and a framework, in particular a suitable framework.

Variable regions for use in the present disclosure will generally be derived from an antibody, which may be generated by any method known in the art.

"Derived from" as employed herein refers to the fact that the sequence employed or a sequence highly similar to the sequence employed was obtained from the original genetic material, such as the light or heavy chain of an antibody.

A "derivative of a naturally occurring domain" as employed herein is intended to refer to where one, two, three, four or five amino acids in a naturally occurring sequence have been replaced or deleted, for example to optimize the properties of the domain such as by eliminating undesirable properties but wherein the characterizing feature(s) of the domain is/are retained. Examples of modifications are those to remove glycosylation sites, GPI anchors, or solvent exposed lysines. These modifications can be achieved by replacing the relevant amino acid residues with a conservative amino acid substitution.

In one embodiment, the antibody molecules of the present disclosure or antibody/fragment components thereof are processed to provide improved affinity for a target antigen or antigens. Such variants can be obtained by a number of affinity maturation protocols including mutating the CDRs (Yang et al J. Mol. Biol., 254, 392-403, 1995), chain shuffling (Marks et al Bio/Technology, 10, 779-783, 1992), use of mutator strains of *E. coli* (Low et al J. Mol. Biol., 250, 359-368, 1996), DNA shuffling (Patten et al Curr. Opin. Biotechnol 8, 724-733, 1997), phage display (Thompson et al J. Mol. Biol., 256, 77-88, 1996) and sexual PCR (Crameri et al Nature, 391, 288-291, 1998). Vaughan *et al* (*supra*) discusses these methods of affinity maturation.

"Highly similar" as employed herein is intended to refer to an amino acid sequence which over its full length is 95% similar or more, such as 96, 97, 98 or 99% similar.

Variable regions for use in the present invention, as described herein above for V_{H} and V_{L} may be from any suitable source and may be for example, fully human or humanised.

In one embodiment the binding domain formed by V_{H} and V_{L} are specific to a first antigen.

In one embodiment the binding domain formed by V₁ is specific to a second antigen.

In one embodiment the binding domain formed by V₂ is specific to a second or third antigen.

In one embodiment the CH₁ domain is a naturally occurring domain 1 from an antibody heavy chain or a derivative thereof. In one embodiment the CH₂ domain is a naturally occurring domain 2 from an antibody heavy chain or a derivative thereof. In one embodiment the CH₃ domain is a naturally occurring domain 3 from an antibody heavy chain or a derivative thereof.

In one embodiment the C_{L} fragment, in the light chain, is a constant kappa sequence or a constant lambda sequence or a derivative thereof.

A derivative of a naturally occurring domain as employed herein is intended to refer to where one, two, three, four or five amino acids in a naturally occurring sequence have been replaced or deleted, for example to optimize the properties of the domain such as by eliminating undesirable properties but wherein the characterizing feature(s) of the domain is/are retained.

In one embodiment one or more natural or engineered inter chain (i.e. inter light and heavy chain) disulphide bonds are present in the functional Fab or Fab' portion of the multi-specific antibody molecule.

In one embodiment a "natural" disulfide bond is present between a CH₁ and C_{L} in the polypeptide chains of:
Formula **(I)** and **(II)**; or Formula **(Ia)** and **(IIa)**; or Formula **(Ib)** and **(IIb).**

When the C_{L} domain is derived from either Kappa or Lambda the natural position for a bond forming cysteine is 214 in human cKappa and cLambda (Kabat numbering 4^{th} edition 1987).

The exact location of the disulfide bond forming cysteine in CH₁ depends on the particular domain actually employed. Thus, for example in human gamma-1 the natural position of the disulfide bond is located at position 233 (Kabat numbering 4^{th} edition 1987). The position of the bond forming cysteine for other human isotypes such as gamma 2, 3, 4, IgM and IgD are known, for example position 127 for human IgM, IgE, IgG2, IgG3, IgG4 and 128 of the heavy chain of human IgD and IgA2B.

Optionally there may be a disulfide bond between the V_{H} and V_{L} of the polypeptides of formula I and II.

In one embodiment the multi-specific antibody according to the disclosure has a disulfide bond in a position equivalent or corresponding to that naturally occurring between CH₁ and C_{L}.

In one embodiment a constant region comprising CH₁ and a constant region such as C_{L} has a disulfide bond which is in a non-naturally occurring position. This may be engineered into the molecule by introducing cysteine(s) into the amino acid chain at the position or positions required. This non-natural disulfide bond is in addition to or as an alternative to the natural disulfide bond present between CH₁ and C_{L}. The cysteine(s) in natural positions can be replaced by an amino acid such as serine which is incapable on forming a disulfide bridge.

Introduction of engineered cysteines can be performed using any method known in the art. These methods include, but are not limited to, PCR extension overlap mutagenesis, site-directed mutagenesis or cassette mutagenesis (see, generally, Sambrook et al Molecular Cloning, A Laboratory Manual, Cold Spring Harbour Laboratory Press, Cold Spring Harbour, NY, 1989; Ausbel et al Current Protocols in Molecular Biology, Greene Publishing & Wiley-Interscience, NY, 1993). Site-directed mutagenesis kits are commercially available, e.g. QuikChange® Site-Directed Mutagenesis kit (Stratagene, La Jolla, CA). Cassette mutagenesis can be performed based on Wells et al 1985, Gene, 34:315-323. Alternatively, mutants can be made by total gene synthesis by annealing, ligation and PCR amplification and cloning of overlapping oligonucleotides.

In one embodiment a disulfide bond between CH₁ and C_{L} is completely absent, for example the interchain cysteines may be replaced by another amino acid, such as serine. Thus in one embodiment there are no inter chain disulphide bonds in the functional Fab or Fab' portion of the multi-specific antibody molecule. Disclosures, such as WO2005/003170, describe how to provide Fab fragments without an inter chain disulphide bond.

In one embodiment the compounds/constructs of the present disclosure comprise a hinge region, for example in the natural location between CH₁ and CH₂. In one embodiment the sequence of the hinge is natural or modified. A natural hinge sequence as employed herein is a sequence occurring naturally in any isotype of antibody (even if that hinge is contained in a different isotype to that in which it occurs naturally). A modified hinge is where 1 to 5 amino acids are replaced, deleted or a combination thereof. In one embodiment the hinge is from an IgG, for example IgG1, IgG2, IgG3 or IgG4.

In one embodiment the compounds/constructs of the present disclosure do not comprise a hinge, for example CH₁ is fused to CH₂ directed or is joined by a linker, for example a linker disclosed herein or based on G4S units.

In one embodiment, CH₃ does not comprise any mutations.

In one embodiment, the antibody molecule does not comprise any 'knobs into holes' mutations.

In one embodiment the antibody molecule of the present disclosure comprises 'knobs into holes' mutations.

"Knobs into holes" or "knobs and holes" as employed herein refers to protuberances at the interface of a first polypeptide and a corresponding cavity in the interface of a second polypeptide, such that the protuberance can be positioned within the cavity in order to promote heteromultimer formation and to hinder homomultimer formation. In this respect, reference is made to US8216805, which describes the use of this method for preparing heteromulitmeric polypeptides such as bi-specific antibodies.

In one embodiment, when p is 1, V₁ is dsFv and q is 0, there are at least two polypeptide chains of formula (**I**).

V₁ represents a dsFv.

V₂ represents a dsFv.

In one embodiment V₂ represents a dsFv and V₁ is absent. In one embodiment V₁ represents a dsFv and V₂ is absent.

"Single chain variable fragment" or "scFv" as employed herein refers to a single chain variable fragment comprising or consisting of a heavy chain variable domain (V_{H}) and a light chain variable domain (V_{L}) which is stabilised by a peptide linker between the V_{H} and V_{L} variable domains. The V_{H} and V_{L} variable domains may be in any suitable orientation, for example the C-terminus of V_{H} may be linked to the N-terminus of V_{L} or the C-terminus of V_{L} may be linked to the N-terminus of V_{H}.

"Disulphide-stabilised single chain variable fragment" or "dsscFv" as employed herein refers to a single chain variable fragment which is stabilised by a peptide linker between the V_{H} and V_{L} variable domain and also includes an inter-domain disulphide bond between V_{H} and V_{L}.

"Disulphide-stabilised variable fragment" or "dsFv" as employed herein refers to a single chain variable fragment which does not include a peptide linker between the V_{H} and V_{L} variable domains and is instead stabilised by an inter-domain disulphide bond between V_{H} and V_{L}.

"Single domain antibody" or "sdAb" as employed herein refers to an antibody fragment consisting of a single monomeric variable antibody domain, such as V_{H} or V_{L}.

In one embodiment p is 1 and q is 0. In one embodiment p is 0 and q is 1.

In one embodiment, the disulfide bond between the variable domains V_{H} and V_{L} of V₁ or the variable domains V_{H} and V_{L} of V₂ is between two of the residues listed below (unless the context indicates otherwise Kabat numbering is employed in the list below). Wherever reference is made to Kabat numbering the relevant reference is Kabat et al 1987, in Sequences of Proteins of Immunological Interest, US Department of Health and Human Services, NIH, USA.

In one embodiment the disulfide bond is in a position selected from the group comprising:
- V_{H}37 + V_{L}95C see for example Protein Science 6, 781-788 Zhu et al (1997);
- V_{H}44 + V_{L}100 see for example; Biochemistry 33 5451-5459 Reiter et al (1994); or Journal of Biological Chemistry Vol. 269 No. 28 pp.18327-18331 Reiter et al (1994); or Protein Engineering, vol.10 no.12 pp.1453-1459 Rajagopal et al (1997);
- V_{H}44 + V_{L}105 see for example J Biochem. 118, 825-831 Luo et al (1995);
- V_{H}45 + V_{L}87 see for example Protein Science 6, 781-788 Zhu et al (1997);
- V_{H}55 + V_{L}101 see for example FEBS Letters 377 135-139 Young et al (1995);
- V_{H}100 + V_{L}50 see for example Biochemistry 29 1362-1367 Glockshuber et al (1990);
- V_{H}100b + V_{L}49;
- V_{H}98 + V_{L} 46 see for example Protein Science 6, 781-788 Zhu et al (1997);
- V_{H}101 + V_{L}46;
- V_{H}105 + V_{L}43 see for example; Proc. Natl. Acad. Sci. USA Vol. 90 pp.7538-7542 Brinkmann et al (1993); or Proteins 19, 35-47 Jung et al (1994),
- V_{H}106 + V_{L}57 see for example FEBS Letters 377 135-139 Young et al (1995)
and a position corresponding thereto in variable region pair located in the molecule.

In one embodiment, the disulphide bond is formed between positions V_{H}44 and V_{L}100 of V₁ or V₂.

The amino acid pairs listed above are in the positions conducive to replacement by cysteines such that disulfide bonds can be formed. Cysteines can be engineered into these desired positions by known techniques. In one embodiment therefore an engineered cysteine according to the present disclosure refers to where the naturally occurring residue at a given amino acid position has been replaced with a cysteine residue.

Introduction of engineered cysteines can be performed using any method known in the art. These methods include, but are not limited to, PCR extension overlap mutagenesis, site-directed mutagenesis or cassette mutagenesis (see, generally, Sambrook et al., Molecular Cloning, A Laboratory Manual, Cold Spring Harbour Laboratory Press, Cold Spring Harbour, NY, 1989; Ausbel et al., Current Protocols in Molecular Biology, Greene Publishing & Wiley-Interscience, NY, 1993). Site-directed mutagenesis kits are commercially available, e.g. QuikChange® Site-Directed Mutagenesis kit (Stratagen, La Jolla, CA). Cassette mutagenesis can be performed based on Wells et al 1985, Gene, 34:315-323. Alternatively, mutants can be made by total gene synthesis by annealing, ligation and PCR amplification and cloning of overlapping oligonucleotides.

Accordingly, in one embodiment, the variable domains V_{H} and V_{L} of each V₁ or V₂ may be linked by a disulfide bond between two cysteine residues, wherein the position of the pair of cysteine residues is independently selected from the group consisting of: V_{H}37 and V_{L}95, V_{H}44 and V_{L}100, V_{H}44 and V_{L}105, V_{H}45 and V_{L}87, V_{H}100 and V_{L}50, V_{H}100b and V_{L}49, V_{H}98 and V_{L}46, V_{H}101 and V_{L}46, V_{H}105 and V_{L}43 and V_{H}106 and V_{L}57.

In one embodiment, the variable domains V_{H} and V_{L} of V₁ or the variable domains V_{H} and V_{L} of V₂ may be linked by a disulfide bond between two cysteine residues, one in V_{H} and one in V_{L}, which is/are outside of the CDRs wherein the position of the pair of cysteine residues is independently selected from the group consisting of V_{H}37 and V_{L}95, V_{H}44 and V_{L}100, V_{H}44 and V_{L}105, V_{H}45 and V_{L}87, V_{H}100 and V_{L}50, V_{H}98 and V_{L}46, V_{H}105 and V_{L}43 and V_{H}106 and V_{L}57.

In one embodiment, the variable domains V_{H} and V_{L} of V₁ are linked by a disulphide bond between two engineered cysteine residues, one at position V_{H}44 and the other at V_{L}100.

In one embodiment, the variable domains V_{H} and V_{L} of V₂ are linked by a disulphide bond between two engineered cysteine residues, one at position V_{H}44 and the other at V_{L}100.

In one embodiment when V₁ is a dsFv, the V_{H} domain of V₁ is attached to X. In one embodiment when V₁ is a dsFv, the V_{L} domain of V₁ is attached to X. In one embodiment when V₂ is a dsFv, the V_{L} domain of V₂ is attached to Y.

In one embodiment X is a bond. In one embodiment Y is a bond.

In one embodiment X is a linker, preferably a peptide linker, for example a suitable peptide for connecting the portions CH₃ and V₁. In one embodiment Y is a linker, preferably a peptide linker, for example a suitable peptide for connecting the portions C_{L} and V₂.

The term "peptide linker" as used herein refers to a peptide with amino acid sequences. A range of suitable peptide linkers will be known to the person of skill in the art.

In one embodiment, the peptide linker may be of synthetic origin, i.e. prepared by synthetic chemistry techniques.

In one embodiment the peptide linker is 50 amino acids in length or less, for example 20 amino acids or less, such as 9, 10, 11, 12, 13 or 14 amino acids in length. In one embodiment the peptide linker is 5-15 amino acids in length.

In one embodiment the linker is selected from a sequence shown in sequence 1 to 67.

In one embodiment the linker is selected from a sequence shown in SEQ ID NO: 1 or SEQ NO:2.

In one embodiment X has the sequence SGGGGSGGGGS (SEQ ID NO: 1).

In one embodiment Y has the sequence SGGGGSGGGGS (SEQ ID NO: 1).

In one embodiment X has the sequence SGGGGTGGGGS (SEQ ID NO: 2).

In one embodiment Y has the sequence SGGGGTGGGGS (SEQ ID NO: 2).

**Table 1. Hinge linker sequences**

| **SEQ ID NO:** | **SEQUENCE** |
|---|---|
| 3 | DKTHTCAA |
| 4 | DKTHTCPPCPA |
| 5 | DKTHTCPPCPATCPPCPA |
| 6 | DKTHTCPPCPATCPPCPATCPPCPA |
| 7 | DKTHTCPPCPAGKPTLYNSLVMSDTAGTCY |
| 8 | DKTHTCPPCPAGKPTHVNVSVVMAEVDGTCY |
| 9 | DKTHTCCVECPPCPA |
| 10 | DKTHTCPRCPEPKSCDTPPPCPRCPA |
| 11 | DKTHTCPSCPA |

**Table 2. Flexible linker sequences**

| **SEQ ID NO:** | **SEQUENCE** |
|---|---|
| 12 | SGGGGSE |
| 13 | DKTHTS |
| 14 | (S)GGGGS |
| 15 | (S)GGGGSGGGGS |
| 16 | (S)GGGGSGGGGSGGGGS |
| 17 | (S)GGGGSGGGGSGGGGSGGGGS |
| 18 | (S)GGGGSGGGGSGGGGSGGGGSGGGGS |
| 19 | AAAGSG-GASAS |
| 20 | AAAGSG-XGGGS-GASAS |
| 21 | AAAGSG-XGGGSXGGGS -GASAS |
| 22 | AAAGSG-XGGGSXGGGSXGGGS-GASAS |
| 23 | AAAGSG- XGGGSXGGGSXGGGSXGGGS-GASAS |
| 24 | AAAGSG-XS-GASAS |
| 25 | PGGNRGTTTTRRPATTTGSSPGPTQSHY |
| 26 | ATTTGSSPGPT |
| 27 | ATTTGS |
| - | GS |
| 28 | EPSGPISTINSPPSKESHKSP |
| 29 | GTVAAPSVFIFPPSD |
| 30 | GGGGIAPSMVGGGGS |
| 31 | GGGGKVEGAGGGGGS |
| 32 | GGGGSMKSHDGGGGS |
| 33 | GGGGNLITIVGGGGS |
| 34 | GGGGVVPSLPGGGGS |
| 35 | GGEKSIPGGGGS |
| 36 | RPLSYRPPFPFGFPSVRP |
| 37 | YPRSIYIRRRHPSPSLTT |
| 38 | TPSHLSHILPSFGLPTFN |
| 39 | RPVSPFTFPRLSNSWLPA |
| 40 | SPAAHFPRSIPRPGPIRT |
| 41 | APGPSAPSHRSLPSRAFG |
| 42 | PRNSIHFLHPLLVAPLGA |
| 43 | MPSLSGVLQVRYLSPPDL |
| 44 | SPQYPSPLTLTLPPHPSL |
| 45 | NPSLNPPSYLHRAPSRIS |
| 46 | LPWRTSLLPSLPLRRRP |
| 47 | PPLFAKGPVGLLSRSFPP |
| 48 | VPPAPVVSLRSAHARPPY |
| 49 | LRPTPPRVRSYTCCPTP- |
| 50 | PNVAHVLPLLTVPWDNLR |
| 51 | CNPLLPLCARSPAVRTFP |

| | |
|---|---|
| (S) is optional in sequences 14 to 18. X is an amino acid. | |

Examples of rigid linkers include the peptide sequences GAPAPAAPAPA (SEQ ID NO: 52), PPPP (SEQ ID NO: 53) and PPP.

In one embodiment the peptide linker is an albumin binding peptide.

Examples of albumin binding peptides are provided in WO2007/106120 and include:

**Table 3**

| **SEQ ID NO:** | **SEQUENCE** |
|---|---|
| 54 | DLCLRDWGCLW |
| 55 | DICLPRWGCLW |
| 56 | MEDICLPRWGCLWGD |
| 57 | QRLMEDICLPRWGCLWEDDE |
| 58 | QGLIGDICLPRWGCLWGRSV |
| 59 | QGLIGDICLPRWGCLWGRSVK |
| 60 | EDICLPRWGCLWEDD |
| 61 | RLMEDICLPRWGCLWEDD |
| 62 | MEDICLPRWGCLWEDD |
| 63 | MEDICLPRWGCLWED |
| 64 | RLMEDICLARWGCLWEDD |
| 65 | EVRSFCTRWPAEKSCKPLRG |
| 66 | RAPESFVCYWETICFERSEQ |
| 67 | EMCYFPGICWM |

Advantageously use of albumin binding peptides as a linker may increase the half-life of the multi-specific antibody molecule.

In one embodiment the linker X and/or Y does not contain a protease cleavage site i.e. an amino acid sequence or motif which is cleaved by a protease. In particular, in one embodiment, linkers X and/or Y do not contain a Furin specific protease cleavage site.

In one embodiment the linker employed in X or in Y is in the range of 4 to 16 amino acids in length, for example multiples of the monomer (G₄S).

The present disclosure also provides sequences which are 80%, 90%, 91%, 92%, 93% 94%, 95% 96%, 97%, 98% or 99% similar to a sequence disclosed herein.

"Identity", as used herein, indicates that at any particular position in the aligned sequences, the amino acid residue is identical between the sequences.

"Similarity", as used herein, indicates that, at any particular position in the aligned sequences, the amino acid residue is of a similar type between the sequences. For example, leucine may be substituted for isoleucine or valine. Other amino acids which can often be substituted for one another include but are not limited to:
- phenylalanine, tyrosine and tryptophan (amino acids having aromatic side chains);
- lysine, arginine and histidine (amino acids having basic side chains);
- aspartate and glutamate (amino acids having acidic side chains);
- asparagine and glutamine (amino acids having amide side chains); and
- cysteine and methionine (amino acids having sulphur-containing side chains).

Degrees of identity and similarity can be readily calculated (Computational Molecular Biology, Lesk, A.M., ed., Oxford University Press, New York, 1988; Biocomputing. Informatics and Genome Projects, Smith, D.W., ed., Academic Press, New York, 1993; Computer Analysis of Sequence Data, Part 1, Griffin, A.M., and Griffin, H.G., eds., Humana Press, New Jersey, 1994; Sequence Analysis in Molecular Biology, von Heinje, G., Academic Press, 1987, Sequence Analysis Primer, Gribskov, M. and Devereux, J., eds., M Stockton Press, New York, 1991, the BLAST™ software available from NCBI (Altschul, S.F. et al., 1990, J. Mol. Biol. 215:403-410; Gish, W. & States, D.J. 1993, Nature Genet. 3:266-272. Madden, T.L. et al 1996, Meth. Enzymol. 266:131-141; Altschul, S.F. et al 1997, Nucleic Acids Res. 25:3389-3402; Zhang, J. & Madden, T.L. 1997, Genome Res. 7:649-656,).

The term "variant" as used herein refers to peptide or protein that contains at least one amino acid sequence or nucleotide sequence alteration as compared to the amino acid or nucleotide sequence of the corresponding wild-type peptide or protein. A variant may comprise at least 80%, or 85%, or 90%, or 95%, or 98% or 99% sequence identity to the corresponding wild-type peptide or protein. However, it is possible for a variant to comprise less than 80% sequence identity, provided that the variant exhibits substantially similar function to its corresponding wild-type peptide or protein.

Antibodies for use in the present invention may be generated by any suitable method known in the art. Antibodies generated against an antigen polypeptide may be obtained, where immunisation of an animal is necessary, by administering the polypeptides to an animal, preferably a non-human animal, using well-known and routine protocols, see for example Handbook of Experimental Immunology, D. M. Weir (ed.), Vol 4, Blackwell Scientific Publishers, Oxford, England, 1986). Many warm-blooded animals, such as rabbits, mice, rats, sheep, cows, camels or pigs may be immunized. However, mice, rabbits, pigs and rats are generally most suitable.

Monoclonal antibodies may be prepared by any method known in the art such as the hybridoma technique (Kohler & Milstein, 1975, Nature, 256:495-497), the trioma technique, the human B-cell hybridoma technique (Kozbor et al 1983, Immunology Today, 4:72) and the EBV-hybridoma technique (Cole et al., Monoclonal Antibodies and Cancer Therapy, pp77-96, Alan R Liss, Inc., 1985).

Antibodies may also be generated using single lymphocyte antibody methods by cloning and expressing immunoglobulin variable region cDNAs generated from single lymphocytes selected for the production of specific antibodies by, for example, the methods described by Babcook, J. et al 1996, Proc. Natl. Acad. Sci. USA 93(15):7843-78481; WO92/02551; WO2004/051268 and WO2004/106377.

The antibodies for use in the present disclosure can also be generated using various phage display methods known in the art and include those disclosed by Brinkman et al. (in J. Immunol. Methods, 1995, 182: 41-50), Ames et al. (J. Immunol. Methods, 1995, 184:177-186), Kettleborough et a. (Eur. J. Immunol. 1994, 24:952-958), Persic et al. (Gene, 1997 187 9-18), Burton et al. (Advances in Immunology, 1994, 57:191-280) and WO90/02809; WO91/10737; WO92/01047; WO92/18619; WO93/11236; WO95/15982; WO95/20401; and US5,698,426; 5,223,409; 5,403,484; 5,580,717; 5,427,908; 5,750,753; 5,821,047; 5,571,698; 5,427,908; 5,516,637; 5,780,225; 5,658,727; 5,733,743; 5,969,108, and WO20011/30305.

In one embodiment the multi-specific molecules according to the disclosure are humanised.

Humanised (which include CDR-grafted antibodies) as employed herein refers to molecules having one or more complementarity determining regions (CDRs) from a non-human species and a framework region from a human immunoglobulin molecule (see, e.g. US5,585,089; WO91/09967). It will be appreciated that it may only be necessary to transfer the specificity determining residues of the CDRs rather than the entire CDR (see for example, Kashmiri et al 2005, Methods, 36, 25-34). Humanised antibodies may optionally further comprise one or more framework residues derived from the non-human species from which the CDRs were derived.

As used herein, the term "humanised antibody molecule" refers to an antibody molecule wherein the heavy and/or light chain contains one or more CDRs (including, if desired, one or more modified CDRs) from a donor antibody (e.g. a murine monoclonal antibody) grafted into a heavy and/or light chain variable region framework of an acceptor antibody (e.g. a human antibody). For a review, see Vaughan et al, Nature Biotechnology, 16, 535-539, 1998. In one embodiment rather than the entire CDR being transferred, only one or more of the specificity determining residues from any one of the CDRs described herein above are transferred to the human antibody framework (see for example, Kashmiri et al 2005, Methods, 36, 25-34). In one embodiment only the specificity determining residues from one or more of the CDRs described herein above are transferred to the human antibody framework. In another embodiment only the specificity determining residues from each of the CDRs described herein above are transferred to the human antibody framework.

When the CDRs or specificity determining residues are grafted, any appropriate acceptor variable region framework sequence may be used having regard to the class/type of the donor antibody from which the CDRs are derived, including mouse, primate and human framework regions. Suitably, the humanised antibody according to the present invention has a variable domain comprising human acceptor framework regions as well as one or more of the CDRs provided herein.

Examples of human frameworks which can be used in the present disclosure are KOL, NEWM, REI, EU, TUR, TEI, LAY and POM (Kabat *et al supra*). For example, KOL and NEWM can be used for the heavy chain, REI can be used for the light chain and EU, LAY and POM can be used for both the heavy chain and the light chain. Alternatively, human germline sequences may be used; these are available at: http://www2.mrc-lmb.cam.ac.uk/vbase/list2.php.

In a humanised antibody molecule of the present disclosure, the acceptor heavy and light chains do not necessarily need to be derived from the same antibody and may, if desired, comprise composite chains having framework regions derived from different chains.

The framework regions need not have exactly the same sequence as those of the acceptor antibody. For instance, unusual residues may be changed to more frequently-occurring residues for that acceptor chain class or type. Alternatively, selected residues in the acceptor framework regions may be changed so that they correspond to the residue found at the same position in the donor antibody (see Reichmann et al 1998, Nature, 332, 323-324). Such changes should be kept to the minimum necessary to recover the affinity of the donor antibody. A protocol for selecting residues in the acceptor framework regions which may need to be changed is set forth in WO91/09967.

Derivatives of frameworks may have 1, 2, 3 or 4 amino acids replaced with an alternative amino acid, for example with a donor residue.
Donor residues are residues from the donor antibody, i.e. the antibody from which the CDRs were originally derived. Donor residues may be replaced by a suitable residue derived from a human receptor framework (acceptor residues). In one embodiment the multi-specific antibodies of the present disclosure are fully human, in particular one or more of the variable domains are fully human.

Fully human molecules are those in which the variable regions and the constant regions (where present) of both the heavy and the light chains are all of human origin, or substantially identical to sequences of human origin, not necessarily from the same antibody. Examples of fully human antibodies may include antibodies produced, for example by the phage display methods described above and antibodies produced by mice in which the murine immunoglobulin variable and optionally the constant region genes have been replaced by their human counterparts eg. as described in general terms in EP0546073, US5,545,806, US5,569,825, US5,625,126, US5,633,425, US5,661,016, US5,770,429, EP0438474 and EP0463151.

In one embodiment the multi-specific antibody molecules of the disclosure are capable of selectively binding two, three or more different antigens of interest.

In one embodiment, antigens of interest bound by the antigen binding domain formed by V_{H}/V_{L}, or V₁ or V₂ are independently selected from a cell-associated protein, for example a cell surface protein on cells such as bacterial cells, yeast cells, T-cells, endothelial cells or tumour cells, and a soluble protein.

Antigens of interest may also be any medically relevant protein such as those proteins upregulated during disease or infection, for example receptors and/or their corresponding ligands. Particular examples of antigens include cell surface receptors such as T cell or B cell signalling receptors, co-stimulatory molecules, checkpoint inhibitors, natural killer cell receptors, Immunoglobulin receptors, TNFR family receptors, B7 family receptors, adhesion molecules, integrins, cytokine/chemokine receptors, GPCRs, growth factor receptors, kinase receptors, tissue-specific antigens, cancer antigens, pathogen recognition receptors, complement receptors, hormone receptors or soluble molecules such as cytokines, chemokines, leukotrienes, growth factors, hormones or enzymes or ion channels, epitopes, fragments and post translationally modified forms thereof.

In one embodiment the antibody protein of the disclosure may be used to functionally alter the activity of the antigen(s) of interest. For example, the antibody fusion protein may neutralize, antagonize or agonise the activity of said antigen, directly or indirectly.

In one embodiment V₁ is specific for human serum albumin. In one embodiment V₂ is specific for human serum albumin.

In one embodiment, an antigen of interest bound by V_{H}/V_{L} or V₁ or V₂ provides the ability to recruit effector functions, such as complement pathway activation and/or effector cell recruitment.

The recruitment of effector function may be direct in that effector function is associated with a cell, said cell bearing a recruitment molecule on its surface. Indirect recruitment may occur when binding of an antigen to an antigen binding domain (such as V₁ or V₂) in the molecule according to present disclosure to a recruitment polypeptide causes release of, for example, a factor which in turn may directly or indirectly recruit effector function, or may be via activation of a signalling pathway. Examples include IL2, IL6, IL8, IFNγ, histamine, C1q, opsonin and other members of the classical and alternative complement activation cascades, such as C2, C4, C3-convertase, and C5 to C9.

As used herein, "a recruitment polypeptide" includes a FcγR such as FcγRI, FcγRII and FcγRIII, a complement pathway protein such as, but without limitation, C1q and C3, a CD marker protein (Cluster of Differentiation marker) or a fragment of any thereof which retains the ability to recruit cell-mediated effector function either directly or indirectly. A recruitment polypeptide also includes immunoglobulin molecules such as IgG1, IgG2, IgG3, IgG4, IgE and IgA which possess effector function.

In one embodiment an antigen binding domain (such as V₁ or V₂) in the multi-specific antibody molecule according to the present disclosure has specificity for a complement pathway protein, with C1q being particularly preferred.

Further, multi-specific antibody molecules of the present disclosure may be used to chelate radionuclides by virtue of a single domain antibody which binds to a nuclide chelator protein. Such fusion proteins are of use in imaging or radionuclide targeting approaches to therapy.

In one embodiment an antigen binding domain within a molecule according to the disclosure (such as V₁ or V₂) has specificity for a serum carrier protein, a circulating immunoglobulin molecule, or CD35/CR1, for example for providing an extended half-life to the antibody fragment with specificity for said antigen of interest by binding to said serum carrier protein, circulating immunoglobulin molecule or CD35/CR1.

As used herein, "serum carrier proteins" include thyroxine-binding protein, transthyretin, α1-acid glycoprotein, transferrin, fibrinogen and albumin, or a fragment of any thereof.

As used herein, a "circulating immunoglobulin molecule" includes IgG1, IgG2, IgG3, IgG4, sIgA, IgM and IgD, or a fragment of any thereof.

CD35/CR1 is a protein present on red blood cells which have a half-life of 36 days (normal range of 28 to 47 days; Lanaro et al 1971, Cancer, 28(3):658-661).

In one embodiment, the antigen of interest for which V_{H}/V_{L} has specificity is a serum carrier protein, such as a human serum carrier.

In one embodiment, the antigen of interest for which V₁ has specificity is a serum carrier protein, such as a human serum carrier.

In one embodiment, the antigen of interest for which V₂ has specificity is a serum carrier protein, such as a human serum carrier.

In one embodiment the multi-specific antibody molecules of the present disclosure are processed to provide improved affinity for a target antigen or antigens. Such variants can be obtained by a number of affinity maturation protocols including mutating the CDRs (Yang et al., J. Mol. Biol., 254, 392-403, 1995), chain shuffling (Marks et al., Bio/Technology, 10, 779-783, 1992), use of mutator strains of *E. coli* (Low et al J. Mol. Biol., 250, 359-368, 1996), DNA shuffling (Patten et al Curr. Opin. Biotechnol., 8, 724-733, 1997), phage display (Thompson et al J. Mol. Biol., 256, 77-88, 1996) and sexual PCR (Crameri et al Nature, 391, 288-291, 1998). Vaughan *et al* (*supra*) discusses these methods of affinity maturation.

Improved affinity as employed herein in this context refers to an improvement over the starting molecule.

If desired an antibody molecule for use in the present disclosure may be conjugated to one or more effector molecule(s). It will be appreciated that the effector molecule may comprise a single effector molecule or two or more such molecules so linked as to form a single moiety that can be attached to the antibodies of the present invention. Where it is desired to obtain an antibody fragment linked to an effector molecule, this may be prepared by standard chemical or recombinant DNA procedures in which the antibody fragment is linked either directly or via a coupling agent to the effector molecule. Techniques for conjugating such effector molecules to antibodies are well known in the art (see, Hellstrom et al Controlled Drug Delivery, 2nd Ed., Robinson et al eds., 1987, pp. 623-53; Thorpe et al 1982 , Immunol. Rev., 62:119-58 and Dubowchik et al 1999, Pharmacology and Therapeutics, 83, 67-123). Particular chemical procedures include, for example, those described in WO93/06231, WO92/22583, WO89/00195, WO89/01476 and WO03031581. Alternatively, where the effector molecule is a protein or polypeptide the linkage may be achieved using recombinant DNA procedures, for example as described in WO86/01533 and EP0392745.

The term "effector molecule" as used herein includes, for example, biologically active proteins, for example enzymes, other antibody or antibody fragments, synthetic or naturally occurring polymers, nucleic acids and fragments thereof e.g. DNA, RNA and fragments thereof, radionuclides, particularly radioiodide, radioisotopes, chelated metals, nanoparticles and reporter groups such as fluorescent compounds or compounds which may be detected by NMR or ESR spectroscopy.

Other effector molecules may include chelated radionuclides such as 111In and 90Y, Lu177, Bismuth213, Californium252, Iridium192 and Tungsten188/Rhenium188; or drugs such as but not limited to, alkylphosphocholines, topoisomerase I inhibitors, taxoids and suramin.

Other effector molecules include proteins, peptides and enzymes. Enzymes of interest include, but are not limited to, proteolytic enzymes, hydrolases, lyases, isomerases, transferases. Proteins, polypeptides and peptides of interest include, but are not limited to, immunoglobulins, toxins such as abrin, ricin A, pseudomonas exotoxin, or diphtheria toxin, a protein such as insulin, α-interferon, β-interferon, nerve growth factor, platelet derived growth factor or tissue plasminogen activator, a thrombotic agent or an anti-angiogenic agent, e.g. angiostatin or endostatin, or, a biological response modifier such as a lymphokine, interleukin-1 (IL-1), interleukin-2 (IL-2), granulocyte macrophage colony stimulating factor (GM-CSF), granulocyte colony stimulating factor (G-CSF), nerve growth factor (NGF) or other growth factor and immunoglobulins.

Other effector molecules may include detectable substances useful for example in diagnosis. Examples of detectable substances include various enzymes, prosthetic groups, fluorescent materials, luminescent materials, bioluminescent materials, radioactive nuclides, positron emitting metals (for use in positron emission tomography), and non-radioactive paramagnetic metal ions. See generally U.S. Patent No. 4,741,900 for metal ions which can be conjugated to antibodies for use as diagnostics. Suitable enzymes include horseradish peroxidase, alkaline phosphatase, beta-galactosidase, or acetylcholinesterase; suitable prosthetic groups include streptavidin, avidin and biotin; suitable fluorescent materials include umbelliferone, fluorescein, fluorescein isothiocyanate, rhodamine, dichlorotriazinylamine fluorescein, dansyl chloride and phycoerythrin; suitable luminescent materials include luminol; suitable bioluminescent materials include luciferase, luciferin, and aequorin; and suitable radioactive nuclides include 1251, 131I, 111In and 99Tc.

In another embodiment the effector molecule may increase the half-life of the antibody *in vivo,* and/or reduce immunogenicity of the antibody and/or enhance the delivery of an antibody across an epithelial barrier to the immune system. Examples of suitable effector molecules of this type include polymers, albumin, albumin binding proteins or albumin binding compounds such as those described in WO05/117984.

Where the effector molecule is a polymer it may, in general, be a synthetic or a naturally occurring polymer, for example an optionally substituted straight or branched chain polyalkylene, polyalkenylene or polyoxyalkylene polymer or a branched or unbranched polysaccharide, e.g. a homo- or hetero- polysaccharide.

Specific optional substituents which may be present on the above-mentioned synthetic polymers include one or more hydroxy, methyl or methoxy groups.

Specific examples of synthetic polymers include optionally substituted straight or branched chain poly(ethyleneglycol), poly(propyleneglycol) poly(vinylalcohol) or derivatives thereof, especially optionally substituted poly(ethyleneglycol) such as methoxypoly(ethyleneglycol) or derivatives thereof.

Specific naturally occurring polymers include lactose, amylose, dextran, glycogen or derivatives thereof.

"Derivatives" as used herein is intended to include reactive derivatives, for example thiol-selective reactive groups such as maleimides and the like. The reactive group may be linked directly or through a linker segment to the polymer. It will be appreciated that the residue of such a group will in some instances form part of the product as the linking group between the antibody fragment and the polymer.

The size of the polymer may be varied as desired, but will generally be in an average molecular weight range from 500Da to 50000Da, for example from 5000 to 40000Da such as from 20000 to 40000Da. The polymer size may in particular be selected on the basis of the intended use of the product for example ability to localize to certain tissues such as tumors or extend circulating half-life (for review see Chapman, 2002, Advanced Drug Delivery Reviews, 54, 531-545). Thus, for example, where the product is intended to leave the circulation and penetrate tissue, for example for use in the treatment of a tumour, it may be advantageous to use a small molecular weight polymer, for example with a molecular weight of around 5000Da. For applications where the product remains in the circulation, it may be advantageous to use a higher molecular weight polymer, for example having a molecular weight in the range from 20000Da to 40000Da.

Suitable polymers include a polyalkylene polymer, such as a poly(ethyleneglycol) or, especially, a methoxypoly(ethyleneglycol) or a derivative thereof, and especially with a molecular weight in the range from about 15000Da to about 40000Da.

In one embodiment antibodies for use in the present disclosure are attached to poly(ethyleneglycol) (PEG) moieties. In one particular example the antibody is an antibody fragment and the PEG molecules may be attached through any available amino acid side-chain or terminal amino acid functional group located in the antibody fragment, for example any free amino, imino, thiol, hydroxyl or carboxyl group. Such amino acids may occur naturally in the antibody fragment or may be engineered into the fragment using recombinant DNA methods (see for example US5,219,996; US5,667,425; WO98/25971, WO2008/038024). In one embodiment the antibody molecule is a modified Fab fragment wherein the modification is the addition to the C-terminal end of its heavy chain one or more amino acids to allow the attachment of an effector molecule. Suitably, the additional amino acids form a modified hinge region containing one or more cysteine residues to which the effector molecule may be attached. Multiple sites can be used to attach two or more PEG molecules.

Suitably PEG molecules are covalently linked through a thiol group of at least one cysteine residue located in the antibody fragment. Each polymer molecule attached to the modified antibody fragment may be covalently linked to the sulphur atom of a cysteine residue located in the fragment. The covalent linkage will generally be a disulphide bond or, in particular, a sulphur-carbon bond. Where a thiol group is used as the point of attachment appropriately activated effector molecules, for example thiol selective derivatives such as maleimides and cysteine derivatives may be used. An activated polymer may be used as the starting material in the preparation of polymer-modified antibody fragments as described above. The activated polymer may be any polymer containing a thiol reactive group such as an α-halocarboxylic acid or ester, e.g. iodoacetamide, an imide, e.g. maleimide, a vinyl sulphone or a disulphide. Such starting materials may be obtained commercially (for example from Nektar, formerly Shearwater Polymers Inc., Huntsville, AL, USA) or may be prepared from commercially available starting materials using conventional chemical procedures. Particular PEG molecules include 20K methoxy-PEG-amine (obtainable from Nektar, formerly Shearwater; Rapp Polymere; and SunBio) and M-PEG-SPA (obtainable from Nektar, formerly Shearwater).

In one embodiment, an IgG, F(ab')₂, Fab or Fab' in the molecule is PEGylated, i.e. has PEG (poly(ethyleneglycol)) covalently attached thereto, e.g. according to the method disclosed in EP 0948544 or EP1090037 [see also "Poly(ethyleneglycol) Chemistry, Biotechnical and Biomedical Applications", 1992, J. Milton Harris (ed), Plenum Press, New York, "Poly(ethyleneglycol) Chemistry and Biological Applications", 1997, J. Milton Harris and S. Zalipsky (eds), American Chemical Society, Washington DC and "Bioconjugation Protein Coupling Techniques for the Biomedical Sciences", 1998, M. Aslam and A. Dent, Grove Publishers, New York; Chapman, A. 2002, Advanced Drug Delivery Reviews 2002, 54:531-545]. In one embodiment PEG is attached to a cysteine in the hinge region. In one example, a PEG modified Fab fragment has a maleimide group covalently linked to a single thiol group in a modified hinge region. A lysine residue may be covalently linked to the maleimide group and to each of the amine groups on the lysine residue may be attached a methoxypoly(ethyleneglycol) polymer having a molecular weight of approximately 20,000Da. The total molecular weight of the PEG attached to the Fab fragment may therefore be approximately 40,000Da.

Particular PEG molecules include 2-[3-(N-maleimido)propionamido]ethyl amide of N,N'-bis(methoxypoly(ethylene glycol) MW 20,000) modified lysine, also known as PEG2MAL40K (obtainable from Nektar, formerly Shearwater).

Alternative sources of PEG linkers include NOF who supply GL2-400MA2 (wherein m in the structure below is 5) and GL2-400MA (where m is 2) and n is approximately 450:

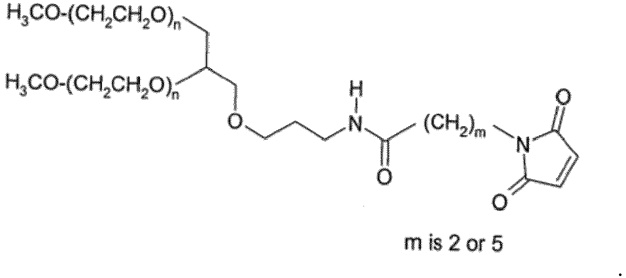

That is to say each PEG is about 20,000Da.

Further alternative PEG effector molecules of the following type: 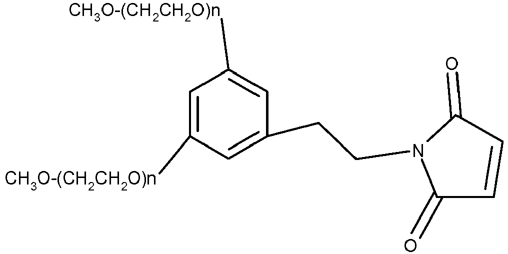 are available from Dr Reddy, NOF and Jenkem.

In one embodiment there is provided an antibody molecule which is PEGylated (for example with a PEG described herein), attached through a cysteine amino acid residue at or about amino acid 226 in the chain, for example amino acid 226 of the heavy chain (by sequential numbering).

In one embodiment there is provided a polynucleotide sequence encoding a molecule of the present disclosure, such as a DNA sequence.

In one embodiment there is provided a polynucleotide sequence encoding one or more, such as two or more or three or more polypeptide components of a molecule of the present disclosure,

In one embodiment the polypeptides do not comprise 'knobs into holes' mutations.

"Mutations" as employed herein refers to nucleic or amino acid sequence comprising one or more naturally occurring or engineered nucleic/amino acid sequence changes compared to the corresponding wild type sequence.

"Knobs into holes" or "knobs and holes" as employed herein refers to protuberances at the interface of a first polypeptide and a corresponding cavity in the interface of a second polypeptide, such that the protuberance can be positioned within the cavity in order to promote heteromultimer formation and to hinder homomultimer formation. In this respect, reference is made to US8,216,805, which describes the use of this method for preparing heteromulitmeric polypeptides such as bi-specific antibodies.

In one embodiment the polynucleotide, such as the DNA is comprised in a vector.

General methods by which the vectors may be constructed, transfection methods and culture methods are well known to those skilled in the art. In this respect, reference is made to "Current Protocols in Molecular Biology", 1999, F. M. Ausubel (ed), Wiley Interscience, New York and the Maniatis Manual produced by Cold Spring Harbor Publishing.

Also provided is a host cell comprising one or more cloning or expression vectors comprising one or more DNA sequences encoding an antibody of the present invention. Any suitable host cell/vector system may be used for expression of the DNA sequences encoding the antibody molecule of the present invention. Bacterial, for example *E. coli,* and other microbial systems may be used or eukaryotic, for example mammalian, host cell expression systems may also be used. Suitable mammalian host cells include CHO, myeloma or hybridoma cells.

As described herein above, the multispecific proteins of the present invention comprise three polypeptide chains, a heavy and light chain and a third 'free' variable domain which binds via a disulphide bond to create a dsFv, which is V₁ or V₂. The present disclosure therefore also provides a process for the production of a multispecific antibody molecule according to the present disclosure comprising culturing a host cell containing a vector or vectors of the present invention under conditions suitable for leading to expression of protein from DNA encoding the three polypeptide chains of the multispecific antibody molecule of the present invention, and isolating the multispecific antibody molecule. In one example of such a process one polypeptide chain consists of a variable domain of V₁ or V₂, i.e the 'free' variable domain that pairs with its complementary V_{H} or V_{L} domain of V₁ or V₂ via a disulphide bond. Also provided are multispecific antibodies obtained or obtainable by this process.

For production of products comprising both heavy and light chains, the cell line may be transfected with two vectors, a first vector encoding a light chain polypeptide and a second vector encoding a heavy chain polypeptide. Alternatively, a single vector may be used, the vector including sequences encoding light chain and heavy chain polypeptides. In one example the cell line may be transfected with three vectors, each encoding a polypeptide chain of an antibody molecule of the present invention. Alternatively a single vector may be used, including sequences encoding three polypeptide chains.

In one embodiment the cell line is transfected with two vectors each one encoding a different polypeptide selected from those disclose herein, for example:
a) a polypeptide chain of formula (**I**):

   **V_{H}-CH₁-CH₂-CH₃-X-(V₁)ₚ;**

   and
b) a polypeptide chain of formula (**II**):

   **V_{L}-C_{L}-Y-(V₂)_{q};**

   wherein:
   - V_{H}: represents a heavy chain variable domain;
   - CH₁: represents a domain of a heavy chain constant region, for example domain 1 thereof;
   - CH₂: represents a domain of a heavy chain constant region, for example domain 2 thereof;
   - CH₃: represents a domain of a heavy chain constant region, for example domain 3 thereof;
   - X: represents a bond or linker;
   - Y: represents a bond or linker;
   - V₁: represents a dsFv;
   - V_{L}: represents a variable domain, for example a light chain variable domain;
   - C_{L}: represents domain from a constant region, for example a light chain constant region domain, such as Ckappa;
   - V₂: represents a dsFv;
   - p: represents 0 or 1;
   - q: represents 0 or 1;
   when p is 1, q is 0, and Y is absent, and when q is 1, p is 0, and X is absent, and
   when q is 1 and V₂ is a dsFv, the VL domain of V₂ is attached to Y, and
   the molecule is provided as a dimer with two heavy chains of formula (I) and two associated light chains of formula (**II**).

In one embodiment, when p is 1, V₁ is dsFv and q is 0, there are at least two polypeptide chains of formula (**I**).

In one embodiment when V₁ is a dsFv and the V_{H} domain of V₁ is attached to X, the cell line may be transfected with a third vector which encodes the V_{L} domain of V₁.

In one embodiment when V₁ is a dsFv and the V_{L} domain of V₁ is attached to X, the cell line may be transfected with a third vector which encodes the V_{H} domain of V₁.

In one embodiment when V₂ is a dsFv and the V_{L} domain of V₂ is attached to Y, the cell line may be transfected with a third vector which encodes the V_{H} domain of V₂.

It will be appreciated that the ratio of each vector transfected into the host cell may be varied in order to optimise expression of the multi-specific antibody product. In one embodiment the ratio of vectors is 1:1:1. It will be appreciated that skilled person is able to find an optimal ratio by routine testing of protein expression levels following transfection.

It will also be appreciated that where two or more, in particular three of more, of the polypeptide components are encoded by a polynucleotide in a single vector the relative expression of each polypeptide component can be varied, if desired, by utilising different promoters for each polynucleotide encoding a polypeptide component of the present disclosure.

In one embodiment the vector comprises a single polynucleotide sequence encoding all three polypeptide chains of the multispecific antibody molecule of the present invention under the control of a single promoter.

In one embodiment the vector comprises a single polynucleotide sequence encoding all three polypeptide chains of the multispecific antibody molecule of the present disclosure wherein each polynucleotide sequence encoding each polypeptide chain is under the control of a different promoter.

The antibodies and fragments according to the present disclosure are expressed at good levels from host cells. Thus the properties of the antibodies and/or fragments appear to be optimised and conducive to commercial processing.

Advantageously, the multi-specific antibody molecules of the present disclosure minimises the amount of aggregation seen after purification and maximise the amount of monomer in the formulations of the construct at pharmaceutical concentrations, for example the monomer may be 50%, 60%, 70% or 75% or more, such as 80, 90, 91, 92, 93, 94, 95, 96, 97, 98 or 99% or more of the protein purified is present as "monomer".

The antibodies of the present disclosure and compositions comprising the same are useful in treatment, for example in the treatment and/or prophylaxis of a pathological condition, in particular as described herein

The pathological condition or disorder, may, for example be selected from the group consisting of infections (viral, bacterial, fungal and parasitic), endotoxic shock associated with infection, arthritis such as rheumatoid arthritis, asthma such as severe asthma, chronic obstructive pulmonary disease (COPD), pelvic inflammatory disease, Alzheimer's Disease, inflammatory bowel disease, Crohn's disease, ulcerative colitis, Peyronie's Disease, coeliac disease, gallbladder disease, Pilonidal disease, peritonitis, psoriasis, vasculitis, surgical adhesions, stroke, Type I Diabetes, lyme disease, meningoencephalitis, autoimmune uveitis, immune mediated inflammatory disorders of the central and peripheral nervous system such as multiple sclerosis, lupus (such as systemic lupus erythematosus) and Guillain-Barre syndrome, Atopic dermatitis, autoimmune hepatitis, fibrosing alveolitis, Grave's disease, IgA nephropathy, idiopathic thrombocytopenic purpura, Meniere's disease, pemphigus, primary biliary cirrhosis, sarcoidosis, scleroderma, Wegener's granulomatosis, other autoimmune disorders, pancreatitis, trauma (surgery), graft-versus-host disease, transplant rejection, heart disease including ischaemic diseases such as myocardial infarction as well as atherosclerosis, intravascular coagulation, bone resorption, osteoporosis, osteoarthritis, periodontitis and hypochlorhydia.

The present disclosure also provides a multi-specific antibody molecule according to the present invention for use in the treatment or prophylaxis of pain, particularly pain associated with inflammation.

The present disclosure further provides a pharmaceutical or diagnostic composition comprising an antibody molecule of the present disclosure in combination with one or more of a pharmaceutically acceptable excipient, diluent or carrier.

The composition will usually be supplied as part of a sterile, pharmaceutical composition that will normally include a pharmaceutically acceptable carrier. A pharmaceutical composition of the present disclosure may additionally comprise a pharmaceutically-acceptable adjuvant.

The present disclosure also provides a process for preparation of a pharmaceutical or diagnostic composition comprising adding and mixing the antibody molecule of the present disclosure together with one or more of a pharmaceutically acceptable excipient, diluent or carrier.

The antibody molecule may be the sole active ingredient in the pharmaceutical or diagnostic composition or may be accompanied by other active ingredients including other antibody ingredients, for example anti- IL-1β, anti-T cell, anti-IFNy or anti-LPS antibodies, or non-antibody ingredients such as xanthines. Other suitable active ingredients include antibodies capable of inducing tolerance, for example, anti-CD3 or anti-CD4 antibodies.

In a further embodiment the antibody, fragment or composition according to the disclosure is employed in combination with a further pharmaceutically active agent.

The pharmaceutical compositions suitably comprise a therapeutically effective amount of the antibody of the invention or multiples thereof

The term "therapeutically effective amount" as used herein refers to an amount of a therapeutic agent needed to treat, ameliorate or prevent a targeted disease or condition, or to exhibit a detectable therapeutic or preventative effect. For any antibody, the therapeutically effective amount can be estimated initially either in cell culture assays or in animal models, usually in rodents, rabbits, dogs, pigs or primates. The animal model may also be used to determine the appropriate concentration range and route of administration. Such information can then be used to determine useful doses and routes for administration in humans.

The precise therapeutically effective amount for a human subject will depend upon the severity of the disease state, the general health of the subject, the age, weight and gender of the subject, diet, time and frequency of administration, drug combination(s), reaction sensitivities and tolerance/response to therapy. This amount can be determined by routine experimentation and is within the judgement of the clinician. Generally, a therapeutically effective amount will be from 0.01 mg/kg to 50 mg/kg, for example 0.1 mg/kg to 20 mg/kg. Alternatively, the dose may be 1 to 500mg per day such as 10 to 100, 200, 300 or 400mg per day. Pharmaceutical compositions may be conveniently presented in unit dose forms containing a predetermined amount of an active agent of the invention.

Compositions may be administered individually to a patient or may be administered in combination (*e.g*. simultaneously, sequentially or separately) with other agents, drugs or hormones.

The dose at which the antibody molecule of the present disclosure is administered depends on the nature of the condition to be treated, the extent of the inflammation present and on whether the antibody molecule is being used prophylactically or to treat an existing condition.

The frequency of dose will depend on the half-life of the antibody molecule and the duration of its effect. If the antibody molecule has a short half-life (e.g. 2 to 10 hours) it may be necessary to give one or more doses per day. Alternatively, if the antibody molecule has a long half-life (e.g. 2 to 15 days) it may only be necessary to give a dosage once per day, once per week or even once every 1 or 2 months.

The pharmaceutically acceptable carrier should not itself induce the production of antibodies harmful to the individual receiving the composition and should not be toxic. Suitable carriers may be large, slowly metabolised macromolecules such as proteins, polypeptides, liposomes, polysaccharides, polylactic acids, polyglycolic acids, polymeric amino acids, amino acid copolymers and inactive virus particles.

Pharmaceutically acceptable salts can be used, for example mineral acid salts, such as hydrochlorides, hydrobromides, phosphates and sulphates, or salts of organic acids, such as acetates, propionates, malonates and benzoates.

Pharmaceutically acceptable carriers in therapeutic compositions may additionally contain liquids such as water, saline, glycerol and ethanol. Additionally, auxiliary substances, such as wetting or emulsifying agents or pH buffering substances, may be present in such compositions. Such carriers enable the pharmaceutical compositions to be formulated as tablets, pills, dragees, capsules, liquids, gels, syrups, slurries and suspensions, for ingestion by the patient.

Suitable forms for administration include forms suitable for parenteral administration, e.g. by injection or infusion, for example by bolus injection or continuous infusion. Where the product is for injection or infusion, it may take the form of a suspension, solution or emulsion in an oily or aqueous vehicle and it may contain formulatory agents, such as suspending, preservative, stabilising and/or dispersing agents. Alternatively, the antibody molecule may be in dry form, for reconstitution before use with an appropriate sterile liquid.

Once formulated, the compositions of the invention can be administered directly to the subject. The subjects to be treated can be animals. However, in one or more embodiments the compositions are adapted for administration to human subjects.

In one embodiment, in formulations according to the present disclosure, the pH of the final formulation is not similar to the value of the isoelectric point of the antibody or fragment, for if the pH of the formulation is 7 then a pI of from 8-9 or above may be appropriate. Whilst not wishing to be bound by theory it is thought that this may ultimately provide a final formulation with improved stability, for example the antibody or fragment remains in solution.

The pharmaceutical compositions of this disclosure may be administered by any number of routes including, but not limited to, oral, intravenous, intramuscular, intra-arterial, intramedullary, intrathecal, intraventricular, transdermal, transcutaneous (for example, see WO98/20734), subcutaneous, intraperitoneal, intranasal, enteral, topical, sublingual, intravaginal or rectal routes. Hyposprays may also be used to administer the pharmaceutical compositions of the invention. Typically, the therapeutic compositions may be prepared as injectables, either as liquid solutions or suspensions. Solid forms suitable for solution in, or suspension in, liquid vehicles prior to injection may also be prepared. Preferably the antibody molecules of the present invention are administered subcutaneously, by inhalation or topically.

Direct delivery of the compositions will generally be accomplished by injection, subcutaneously, intraperitoneally, intravenously or intramuscularly, or delivered to the interstitial space of a tissue. The compositions can also be administered into a specific tissue of interest. Dosage treatment may be a single dose schedule or a multiple dose schedule.

It will be appreciated that the active ingredient in the composition will be an antibody molecule. As such, it will be susceptible to degradation in the gastrointestinal tract. Thus, if the composition is to be administered by a route using the gastrointestinal tract, the composition will need to contain agents which protect the antibody from degradation but which release the antibody once it has been absorbed from the gastrointestinal tract.

A thorough discussion of pharmaceutically acceptable carriers is available in Remington's Pharmaceutical Sciences (Mack Publishing Company, N.J. 1991).

In one embodiment the formulation is provided as a formulation for topical administrations including inhalation.

Suitable inhalable preparations include inhalable powders, metering aerosols containing propellant gases or inhalable solutions free from propellant gases (such as nebulisable solutions or suspensions). Inhalable powders according to the disclosure containing the active substance may consist solely of the abovementioned active substances or of a mixture of the above mentioned active substances with physiologically acceptable excipient.

These inhalable powders may include monosaccharides (e.g. glucose or arabinose), disaccharides (e.g. lactose, saccharose, maltose), oligo- and polysaccharides (e.g. dextranes), polyalcohols (e.g. sorbitol, mannitol, xylitol), salts (e.g. sodium chloride, calcium carbonate) or mixtures of these with one another. Mono- or disaccharides are suitably used, the use of lactose or glucose, particularly but not exclusively in the form of their hydrates.

Particles for deposition in the lung require a particle size less than 10 microns, such as 1-9 microns for example from 0.1 to 5 µm, in particular from 1 to 5 µm. The particle size of the active agent (such as the antibody or antibody fragment) is of primary importance.

The propellent gases which can be used to prepare the inhalable aerosols are known in the art. Suitable propellent gases are selected from among hydrocarbons such as n-propane, n-butane or isobutane and halohydrocarbons such as chlorinated and/or fluorinated derivatives of methane, ethane, propane, butane, cyclopropane or cyclobutane. The abovementioned propellent gases may be used on their own or in mixtures thereof.

Particularly suitable propellent gases are halogenated alkane derivatives selected from among TG 11, TG 12, TG 134a and TG227. Of the abovementioned halogenated hydrocarbons, TG134a (1,1,1,2-tetrafluoroethane) and TG227 (1,1,1,2,3,3,3-heptafluoropropane) and mixtures thereof are particularly suitable.

The propellent-gas-containing inhalable aerosols may also contain other ingredients such as cosolvents, stabilisers, surface-active agents (surfactants), antioxidants, lubricants and means for adjusting the pH. All these ingredients are known in the art.

The propellant-gas-containing inhalable aerosols may contain up to 5 % by weight of active substance. Aerosols contain, for example, 0.002 to 5 % by weight, 0.01 to 3 % by weight, 0.015 to 2 % by weight, 0.1 to 2 % by weight, 0.5 to 2 % by weight or 0.5 to 1 % by weight of active.

Alternatively topical administrations to the lung may also be by administration of a liquid solution or suspension formulation, for example employing a device such as a nebulizer, for example, a nebulizer connected to a compressor (e.g., the Pari LC-Jet Plus(R) nebulizer connected to a Pari Master(R) compressor manufactured by Pari Respiratory Equipment, Inc., Richmond, Va.).

In one embodiment the formulation is provided as discrete ampoules containing a unit dose for delivery by nebulisation.

In one embodiment the antibody is supplied in lyophilised form, for reconstitutions or alternatively as a suspension formulation.

The antibody of the present disclosure can be delivered dispersed in a solvent, e.g., in the form of a solution or a suspension. It can be suspended in an appropriate physiological solution, e.g., physiological saline, a pharmacologically acceptable solvent or a buffered solution. Buffered solutions known in the art may contain 0.05 mg to 0.15 mg disodium edetate, 8.0 mg to 9.0 mg NaCl, 0.15 mg to 0.25 mg polysorbate, 0.25 mg to 0.30 mg anhydrous citric acid, and 0.45 mg to 0.55 mg sodium citrate per 1 ml of water so as to achieve a pH of about 4.0 to 5.0. As mentioned *supra* a suspension can made, for example, from lyophilised antibody.

The composition may, optionally, comprise further molecules capable of altering the characteristics of the population of antibodies thereby, for example, reducing, stabilizing, delaying, modulating and/or activating the function of the antibodies.

The therapeutic suspensions or solution formulations can also contain one or more excipients. Excipients are well known in the art and include buffers (e.g., citrate buffer, phosphate buffer, acetate buffer and bicarbonate buffer), amino acids, urea, alcohols, ascorbic acid, phospholipids, proteins (e.g., serum albumin), EDTA, sodium chloride, liposomes, mannitol, sorbitol, and glycerol. Solutions or suspensions can be encapsulated in liposomes or biodegradable microspheres. The formulation will generally be provided in a substantially sterile form employing sterile manufacture processes.

This may include production and sterilization by filtration of the buffered solvent solution used for the formulation, aseptic suspension of the antibody in the sterile buffered solvent solution, and dispensing of the formulation into sterile receptacles by methods familiar to those of ordinary skill in the art.

Nebulisable formulation according to the present disclosure may be provided, for example, as single dose units (e.g., sealed plastic containers or vials) packed in foil envelopes. Each vial contains a unit dose in a volume, e.g., 2 ml, of solvent/solution buffer.

The antibodies of the present disclosure are thought to be suitable for delivery via nebulisation.

It is also envisaged that the antibody of the present invention may be administered by use of gene therapy. In order to achieve this, DNA sequences encoding the heavy and light chains of the antibody molecule under the control of appropriate DNA components are introduced into a patient such that the antibody chains are expressed from the DNA sequences and assembled *in situ.*

In one embodiment there is provided a process for purifying a multi-specific antibody (in particular an antibody or fragment according to the invention).

In one embodiment there is provided a process for purifying a multi-specific antibody (in particular an antibody or fragment according to the invention) comprising the steps: performing anion exchange chromatography in non-binding mode such that the impurities are retained on the column and the antibody is maintained in the unbound fraction. The step may, for example be performed at a pH about 6-8.

The process may further comprise an initial capture step employing cation exchange chromatography, performed for example at a pH of about 4 to 5.

The process may further comprise of additional chromatography step(s) to ensure product and process related impurities are appropriately resolved from the product stream.

The purification process may also comprise of one or more ultra-filtration steps, such as a concentration and diafiltration step.

Purified form as used *supra* is intended to refer to at least 90% purity, such as 91, 92, 93, 94, 95, 96, 97, 98, 99% w/w or more pure.

Substantially free of endotoxin is generally intended to refer to an endotoxin content of 1 EU per mg antibody product or less such as 0.5 or 0.1 EU per mg product.

Substantially free of host cell protein or DNA is generally intended to refer to host cell protein and/or DNA content 400µg per mg of antibody product or less such as 100µg per mg or less, in particular 20µg per mg, as appropriate.

The antibody molecule of the present invention may also be used in diagnosis, for example in the *in vivo* diagnosis and imaging.

"Comprising" in the context of the present specification is intended to meaning including. Where technically appropriate, embodiments of the invention may be combined. Embodiments are described herein as comprising certain features/elements. The disclosure also extends to separate embodiments consisting or consisting essentially of said features/elements.

Any embodiments specifically and explicitly recited herein may form the basis of a disclaimer either alone or in combination with one or more further embodiment.

The present disclosure is further described by way of illustration only in the following examples, which refer to the accompanying Figures, in which:

### Brief Description of the Figures

- **Figure 1**: shows various multi-specific antibody constructs of the present disclosure. IgG-dsFv formats.
- **Figure 2**: shows SDS-PAGE analysis of various protein-A purified Heavy Chain (HC) linked constructs of the present disclosure.
**(A)**Non-reduced samples. **(B)** Reduced samples.
- **Figure 3**: shows SDS-PAGE analysis of various protein-A purified Light Chain (LC) linked constructs of the present disclosure.
**(A)**Non-reduced samples. **(B)** Reduced samples.

### EXAMPLES

Antibody/fragments to ANTIGEN 1 are labelled #1
Antibody/fragments to ANTIGEN 2 are labelled #2
Antibody/fragments to ANTIGEN 3 are labelled #3
Antibody/fragments to ANTIGEN 4 are labelled #4

### EXAMPLE 1 - Generation of antibody molecules of the present disclosure (IgG-dsFv) Construction of plasmids for expression in mammalian cells

### For IgG#2-dsFv#1 antibody constructs

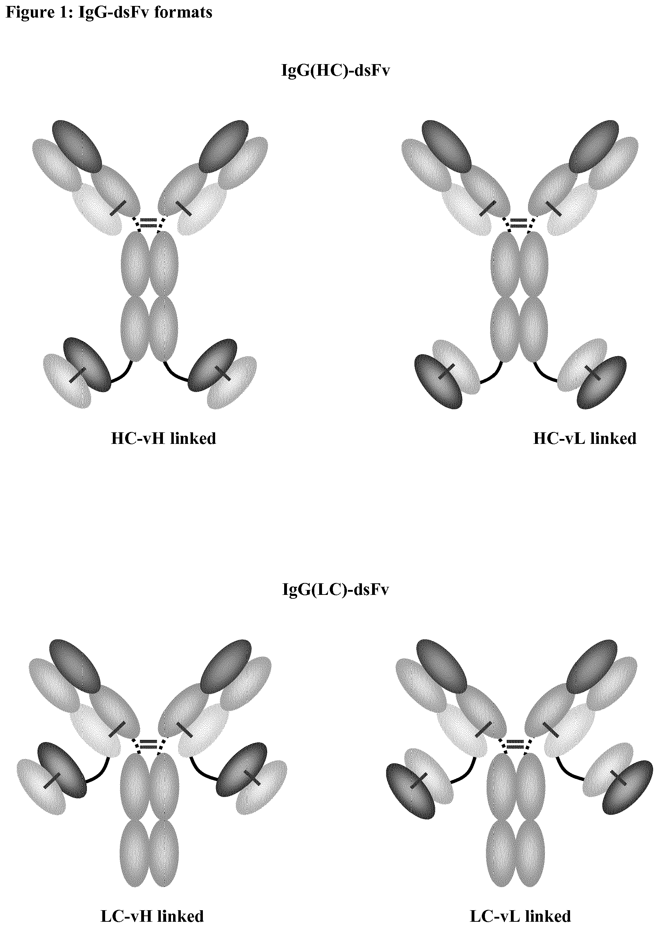

Plasmids for the expression of IgG#2-dsFv#1 (see Figure 1), were constructed by fusing #1vL or #1vH to the C-terminus of the Km3 allotype human kappa constant region of the #2 light chain using the flexible linker SGGGGSGGGGS [also referred to herein as S, 2xG4S] (SEQ ID NO: 1), or by fusing #1vL or #1vH to the C-terminus of the, γ1 isotype human gamma-1 CH3 constant region of the #2 heavy chain using the flexible linker SGGGGSGGGGS (SEQ ID NO: 1). In addition, point mutations were introduced into the DNA sequences at selected residues in the framework region of both #1vL and #1vH. The mutations (heavy chain G44C and light chain G100C) were introduced to create an interchain disulphide bond between the heavy and light chains of the #1Fv.

Gene fragments encoding the #1dsvL free domain and #1dsvH free domain were manufactured chemically and fused to IgG#2 as detailed above to generate:
IgG#2 Light-(SGGGGSGGGGS)-dsvL#1 **[Plasmid A]**;
IgG#2 Light-(SGGGGSGGGGS)-dsvH#1 **[Plasmid B]**;
IgG#2 Heavy-(SGGGGSGGGGS)-dsvL#1 **[Plasmid C]**; and
IgG#2 Heavy-(SGGGGSGGGGS)-dsvH#1 **[Plasmid D]**.

### For IgG#2-dsscFv#1 and IgG#2-scFv#1 antibody constructs

Plasmids for the expression of IgG#2-dsscFv#1 and IgG#2-scFv#1 were constructed as follows. A single chain Fv (scFv) was constructed by linking the N-terminus of #1vL to the C-terminus of #1vH via the flexible linker GGGGSGGGGSGGGGSGGGGS [also referred herein as 4xG4S] (SEQ ID NO: 68). To generate dsscFv, point mutations were introduced into the DNA sequence at framework residues G100C in #1vL and G44C in #1vH to make the disulphide linked scFv (dsscFv).

Gene fragments encoding scFv#1 and dsscFv#1 were manufactured chemically and fused to the C-terminus of either the Km3 allotype human kappa constant region of the #2 light chain using the flexible linker SGGGGSGGGGS (SEQ ID NO: 1) to generate:
#2 Light-(SGGGGSGGGGS)-dsscFv#1(dsvH-4xG4S-dsvL) **[Plasmid E1]**;
#2 Light-(SGGGGSGGGGS)-dsscFv#1(dsvL-4xG4S-dsvH) **[Plasmid E2]**;
#2 Light-(SGGGGSGGGGS)-scFv#1(vH-4xG4S-vL) **[Plasmid F1]**; and
#2 Light-(SGGGGSGGGGS)-scFv#1(vL-4xG4S-vH) **[Plasmid F2]**;
or to the γ1 isotype human gamma-1 CH₃ constant region of the #2 heavy chain using the flexible linker SGGGGSGGGGS (SEQ ID NO: 1) to generate:
#2 Heavy-(SGGGGSGGGGS)-dsscFv#1(dsvH-4xG4S-dsvL) **[Plasmid G1]**;
#2 Heavy-(SGGGGSGGGGS)-dsscFv#1(dsvL-4xG4S-dsvH) **[Plasmid G2]**;
#2 Heavy-(SGGGGSGGGGS)-scFv#1(vH-4xG4S-vL) **[Plasmid H1]**; and
#2 Heavy-(SGGGGSGGGGS)-scFv#1(vL-4xG4S-vH) **[Plasmid H2]**.

All IgG fusion formats were cloned into mammalian expression vectors under the control of the HCMV-MIE promoter and SV40E polyA sequence. The IgG#2 light chain **[Plasmid J],** IgG#2 heavy chain **[Plasmid I],** dsvL#1 free domain **[Plasmid K]** and dsvH#1 free domain **[Plasmid L]** were manufactured chemically and individually cloned into mammalian expression vectors under the control of the HCMV-MIE promoter and SV40E polyA sequence.

### HEK293 expression of:

IgG#2(LC)-dsFv#1, IgG#2(HC)-dsFv#1, IgG#2(LC)-dsscFv#1, IgG#2(HC)-dsscFv#1, IgG#2(LC)-scFv#1 and IgG#2(HC)-scFv#1.

HEK293 cells were transfected with the relevant plasmids using Invitrogen's 293fectin transfection reagent according to the manufacturer's instructions. Plasmids were mixed as shown in Table 4 to express the different constructs:

**Table 4**

| **Antibody Construct** | **Plasmids used** |
|---|---|
| IgG#2(LC)-dsFv#1 (LC-vH linked) | 1. **Plasmid B** |
| | 2. **Plasmid I** |
| | 3. **Plasmid K** |
| IgG#2(LC)-dsFv#1 (LC-vL linked) | 1. **Plasmid A** |
| | 2. **Plasmid I** |
| | 3. **Plasmid L** |
| IgG#2(HC)-dsFv#1 (HC-vH linked) | 1. **Plasmid D** |
| | 2. **Plasmid J** |
| | 3. **Plasmid K** |
| IgG#2(HC)-dsFv#1 (HC-vL linked) | 1. **Plasmid C** |
| | 2. **Plasmid J** |
| | 3. **Plasmid L** |
| IgG#2(LC)-dsHLscFv#1 (LC-dsscFv linked) | 1. **Plasmid I** |
| | 2. **Plasmid E1** |
| IgG#2(LC)-HLscFv#1 (LC-scFv linked) | 1. **Plasmid I** |
| | 2. **Plasmid F1** |
| IgG#2(LC)-dsLHscFv#1 (LC-dsscFv linked) | 1. **Plasmid I** |
| | 2. **Plasmid E2** |
| IgG#2(LC)-LHscFv#1 (LC-scFv linked) | 1. **Plasmid I** |
| | 2. **Plasmid F2** |
| IgG#2(HC)-dsHLscFv#1 (HC-dsscFv linked) | 1. **Plasmid J** |
| | 2. **Plasmid G1** |
| IgG#2(HC)-HLscFv#1 (HC-scFv linked) | 1. **Plasmid J** |
| | 2. **Plasmid H1** |
| IgG#2(HC)-dsLHscFv#1 (HC-dsscFv linked) | 1. **Plasmid J** |
| | 2. **Plasmid G2** |
| IgG#2(HC)-LHscFv#1 (HC-scFv linked) | 1. **Plasmid J** |
| | 2. **Plasmid H2** |

The ratio of the plasmids used for the transfections varied: for the 2 plasmid combinations the ratio was 1:1, whereas for the 3 plasmid combinations different ratios were tested. A total of 50µg plasmid DNA was incubated with 125µl 293fectin + 4.25ml Optimem media for 20mins at RT. The mixture was then added to 50ml HEK293 cells in suspension at 1 x 10⁶ cells/ml and incubated with shaking at 37°C. Supernatants were harvested on day 10 by centrifugation at 1500g to remove cells and the supernatant was passed through a 0.22µm filter. Expression level was determined by Protein-G HPLC.

Table 5 shows the results of the Protein-G HPLC. As can be seen, the levels of expression of all the constructs were comparable to each other, covering a range of 7-22µg/ml. The IgG-dsFv expressed at 7-22µg/ml, the IgG-dsscFv expressed at 9-17µg/ml and the IgG-scFv expressed at 10-16µg/ml. There have been reports in the literature that the expression of Fv regions that lack either a linker between the vL and vH or a dimerisation motif to bring the vL and vH together have substantially lower expression levels than linked Fvs. Surprisingly, this was not observed in this data where there was no significant difference observed between the best expression of each type of construct. The vL-linked proteins containing an unlinked vH displayed higher expression levels (10-22µg/ml) compared to the vH-linked proteins containing an unlinked vL (7-8µg/ml).

**Table 5**

| **Antibody Construct** | **Expression level (µg/ml)** |
|---|---|
| IgG#2(LC)-dsFv#1 (LC-vH linked)(ratio 1:1:1 LC-vH:HC:vL) | 7 |
| IgG#2(LC)-dsFv#1 (LC-vH linked) (ratio 1:1:2 LC-vH:HC:vL) | 8 |
| IgG#2(LC)-dsFv#1 (LC-vL linked)(ratio 1:1:1 LC-vL:HC:vH) | 22 |
| IgG#2(LC)-dsFv#1 (LC-vL linked) (ratio 1:1:2 LC-vL:HC:vH) | 17 |
| IgG#2(HC)-dsFv#1 (HC-vH linked)(ratio 1:1:1 HC-vH:LC:vL) | 7 |
| IgG#2(HC)-dsFv#1 (HC-vH linked) (ratio 1:1:2 HC-vH:LC:vL) | 8 |
| IgG#2(HC)-dsFv#1 (HC-vL linked)(ratio 1:1:1 HC-vL:LC:vH) | 13 |
| IgG#2(HC)-dsFv#1 (HC-vL linked) (ratio 1:1:2 HC-vL:LC:vH) | 10 |
| IgG#2(LC)-dsHLscFv#1 (LC-dsscFv linked) | 17 |
| #2IgG(LC)-HLscFv#1 (LC-scFv linked) | 14 |
| IgG#2(LC)-dsLHscFv#1 (LC-dsscFv linked) | 15 |
| IgG#2(LC)-LHscFv#1 (LC-scFv linked) | 16 |
| IgG#2(HC)-dsHLscFv#1 (HC-dsscFv linked) | 10 |
| IgG#2(HC)-HLscFv#1 (HC-scFv linked) | 10 |
| IgG#2(HC)-dsLHscFv#1 (HC-dsscFv linked) | 9 |
| IgG#2(HC)-LHscFv#1 (HC-scFv linked) | 11 |

### Protein-A purification of HEK293 expressed IgG#2-dsFv#1, IgG#2-dsscFv#1 and IgG#2-scFv#1

The ∼50ml HEK293 supernatants were concentrated ∼25 fold to ∼2ml using 30kDa molecular weight cut off centrifugation concentrators. The concentrated supernatants were applied to a 1ml HiTrap MabSelectSure column (GE Healthcare) equilibrated in 20mM phosphate, 40mM NaCl pH7.4. The column was washed with 20mM phosphate, 40mM NaCl pH7.4 and the bound material was eluted with 0.1M citrate, pH3.4. The elution peak was collected and pH adjusted to ∼pH7.0 with 2M Tris/HCl pH8.5. The pH adjusted elutions were concentrated and buffer exchanged into PBS pH7.4 using 30kDa molecular weight cut off centrifugation concentrators.

### Example 2 - Analysis of IgG-dsFv molecules

### SDS-PAGE analysis of Protein-A purified, HEK293 expressed IgG#2-dsFv#1, IgG#2-dsscFv#1 and IgG#2-scFv#1

Samples (2µg) were diluted with PBS to a volume of 9.75µl to which 3.75µl 4xLDS sample buffer and 1.5µl 100 mM N- ethylmaleimide (non-reduced samples) or 1.5µl 10x NuPAGE reducing agent (reduced samples) was added. The samples were vortexed, incubated at 70°C for 10 minutes, cooled and centrifuged at 12500 rpm for 30 seconds. The prepared samples were loaded onto a 4-20% acrylamide Tris/Glycine SDS gel and run for ∼100 minutes at 125V, constant voltage. SeeBluePlus2 (Life Technologies) molecular weight ladder was used. The gels were stained with Instant Blue protein stain (Expedeon) and destained with distilled water.

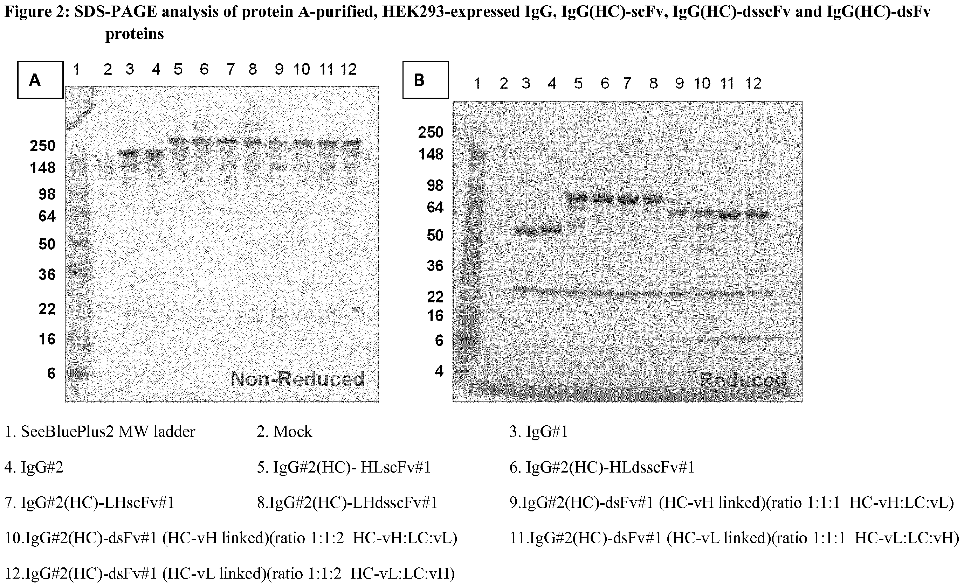

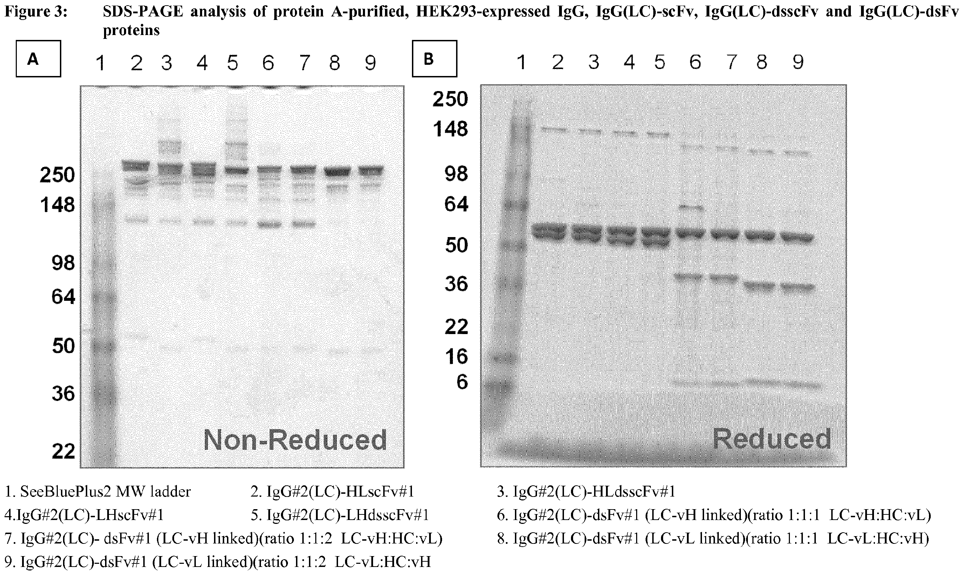

The results of the SDS-PAGE are shown in Figures 2 and 3. The expected band sizes after reducing and non-reducing SDS-PAGE are indicated in Table 6. IgG formats were run alongside for size comparison.

**Table 6:**

| **Expected band sizes after SDS-PAGE (kDa)** | | | | | |
|---|---|---|---|---|---|
| **(H=heavy chain, L=light chain, +/reducing agent)** | | | | | |
| | - Red | + Red | | - Red | + Red |
| **IgG(HC)-scFv** | ∼201 | H∼77 L∼24 | **IgG-(HC)dsFv** | ∼201 | H∼64+12 L∼24 |
| **IgG(LC)-scFv** | ∼201 | H∼50 L∼50 | **IgG-(LC)dsFv** | ∼201 | H∼50 L∼37+12 |
| **IgG** | ∼147 | H∼50 L∼24 | | | |

| | | | | | |
|---|---|---|---|---|---|
| For IgG, the non-reducing gel was expected to show a band at ∼147kDa, whilst the reducing gels were expected to show bands at ∼50kDa and 24kDa with roughly twice the staining in the upper band. For IgG(HC)-scFv and IgG(HC)-dsscFv, the non-reducing gel was expected to show a band at ∼201kDa, whilst the reducing gel was expected to show bands at ∼77kDa and 24kDa with roughly three times the staining in the upper band. For IgG(HC)-dsFv, the non-reducing gel was expected to show a band at ∼201kDa, whilst the reducing gel was expected to show bands at ∼64kDa, 24kDa and 12kDa with staining roughly in the ratio 3:2:1 upper to lower band. For IgG(LC)-scFv and IgG(LC)-dsscFv, the non-reducing gel was expected to show a band at ∼201kDa, whilst the reducing gel was expected to show a doublet at ∼50kDa with roughly equivalent staining in both bands. For IgG(LC)-dsFv, the non-reducing gel was expected to show a band at ∼201kDa, whilst the reducing gel was expected to show bands at ∼50kDa, 37kDa and 12kDa with staining roughly in the ratio 3:2:1 upper to lower band. | | | | | |

The reducing SDS-PAGE gels showed banding patterns which indicated that the constructs were being expressed correctly in terms of both migration position and staining intensity. The only exceptions were IgG#2(HC)-dsFv#1 (HC-vH linked) (transfection ratio 1:1:2 HC-vH:LC:vL), which contained extra bands at ∼55kDa and ∼45kDa (Figure 2B, lane 10), and IgG#2(LC)-dsFv#1 (LC-vH linked)(ratio 1:1:1 LC-vH:HC:vL) (Figure 3B, lane 6), which contained an extra band at ∼65kDa. However, similar extra banding was also observed in the parental IgG#2-scFv#1 (HC-HLscFv) protein (extra banding at ∼65kDa and ∼55kDa, Figure 2B, lane 5) and in the parental IgG#2-dsscFv#1 (LC-HLscFv) protein (extra banding at ∼65kDa, Figure 3B, lane 3).

So whilst the HC-vH linked proteins and LC-vH linked proteins had additional bands that may have been consistent with some minor degradation/aggregation, the HC-vL linked proteins and LC-vL linked proteins displayed the expected banding patterns. Non-reducing SDS-PAGE gels showed banding patterns >200kDa for IgG-dsscFv that were consistent with multimerisation (Figure 2A, lanes 6 and 8, Figure 3A, lanes 3 and 5) but these high molecular weight species were absent in all the IgG-dsFv samples (Figure 2A, lanes 9-12, Figure 3A, lanes 6-9).

### S200 SE-HPLC analysis of Protein-A purified, HEK293 expressed, IgG#2-dsFv#1 and IgG#2-dsscFv#1

10µg purified protein samples (100µl of 0.1 mg/ml stock diluted in PBS) were injected onto a Superdex 200 10/300 GL Tricorn column (GE Healthcare) 3 days post-purification and developed with an isocratic gradient of PBS pH7.4 at 1ml/min, with continuous detection by absorbance at 280nm.

After Protein-A purification the IgG#2(HC)-dsscFv#1 sample was 64-75% monomer, the IgG#2(LC)-dsscFv#1 sample was 53-54% monomer; whereas the IgG#2(HC)-dsFv#1 (HC-vH linked) was 91-92% monomer, the IgG#2(HC)-dsFv#1 (HC-vL linked) was 97-99% monomer, the IgG#2(LC)-dsFv#1 (LC-vH linked) was 60-76% monomer and the IgG#2(LC)-dsFv#1 (LC-vL linked) was 100% monomer.

The Fv#1 is known to be particularly prone to multimerisation. Given that IgG-dsscFv multimers are physically joined by the dsscFv linker, by replacing dsscFv#1 with dsFv#1 (which lacks a scFv linker), there was a significant increase in the level of monomer obtained (in some cases 100%). Depending on the propensity of the Fv to multimerise, in some instances it would be advantageous to use a dsFv instead of a dsscFv. Absence of the linker between vL and vH removes the possibility of inappropriate intermolecular domain interactions and hence reduces the propensity to form multimers and aggregates during and after expression. The presence of the disulphide bond with the dsFv ensures that the product is stable.

## Claims

1. A multi-specific antibody molecule comprising or consisting of:
a) a polypeptide chain of formula (**I**):
**V_{H}-CH₁-CH₂-CH₃-X-(V₁)ₚ**;
and
b) a polypeptide chain of formula (II):
**V_{L}-C_{L}-Y-(V₂)_{q}**;
wherein:
V_{H} represents a heavy chain variable domain;
CH₁ represents a domain of a heavy chain constant region, for example domain 1 thereof;
CH₂ represents a domain of a heavy chain constant region, for example domain 2 thereof;
CH₃ represents a domain of a heavy chain constant region, for example domain 3 thereof;
X represents a bond or linker;
Y represents a bond or linker;
V₁ represents a dsFv;
V_{L} represents a variable domain, for example a light chain variable domain;
C_{L} represents a domain from a constant region, for example a light chain constant region domain, such as Ckappa;
V₂ represents a dsFv;
p represents 0 or 1;
q represents 0 or 1;
wherein at least one of p or q is 1, and
when p is 1, q is 0, and Y is absent, and
when q is 1, p is 0, and X is absent, and
when q is 1 and V₂ is a dsFv, the VL domain of V₂ is attached to Y, and
the molecule is provided as dimer with two heavy chains of formula **(I)** and two associated light chains of formula **(II).**

2. A multi-specific antibody molecule according to claim 1, wherein p is 1.

3. A multi-specific antibody molecule according to claim 1, wherein q is 1.

4. A multi-specific antibody molecule according to claim 1 or 2, wherein X is a peptide linker.

5. A multi-specific antibody molecule according to claim 4, wherein X has the sequence of SEQ ID NO: 1 or SEQ ID NO: 2.

6. A multi-specific antibody molecule according to claim 1 or 2, wherein X is a bond.

7. A multi-specific antibody molecule according to claim 1 or 3, wherein Y is a peptide linker.

8. A multi-specific antibody molecule according to claim 7, wherein Y has the sequence of SEQ ID NO: 1 or SEQ ID NO: 2.

9. A multi-specific antibody molecule according to claim 1 or 3, wherein Y is a bond.

10. A multi-specific antibody molecule according to any one of claims 1 to 9, wherein an intra-variable region disulfide bond in V₁ or V₂ is independently selected from the group comprising or consisting of: V_{H}37 and V_{L}95, V_{H}44 and V_{L}100, V_{H}44 and V_{L}105, V_{H}45 and V_{L}87, V_{H}100 and V_{L}50, V_{H}100b and V_{L}49, V_{H}98 and V_{L}46, V_{H}101 and V_{L}46, V_{H}105 and V_{L}43 and V_{H}106 and V_{L}57, wherein the V_{H} and V_{L} values are independently within a given V₁ or V₂, for example V_{H}44 and V_{L}100, and wherein the numbering is according to Kabat.

11. A multi-specific antibody molecule according to claim 10, wherein the intra-variable region disulfide bond in V₁ or V₂ is in position V_{H}44 and V_{L}100.

12. A polynucleotide encoding a multi-specific antibody molecule according to any one of claims 1 to 11.

13. A vector comprising a polynucleotide defined in claim 12.

14. A host cell comprising the polynucleotide or vector of claim 12 or 13 respectively.

15. A host cell comprising three vectors each vector comprising a polynucleotide encoding a different polypeptide chain of a multi-specific antibody molecule according to any one of claims 1 to 11.

16. A process comprising expressing a multi-specific antibody molecule from a host cell defined in claim 14 or 15.

17. A pharmaceutical composition comprising a multi-specific antibody molecule according to any one of claims 1 to 11 and at least one excipient.

18. A multi-specific antibody molecule according to any one of claims 1 to 11 or a pharmaceutical composition according to claim 17, for use in treatment.

## Patentansprüche

1. Multispezifisches Antikörpermolekül, das
a) eine Polypeptidkette der Formel (**I**)**:**
V_{H}**-CH₁-CH₂CH₃-X-(**V₁**)**;
und
b) eine Polypeptidkette der Formel (**II**):
V_{L}**-C_{L}-Y-(**V₂**)**;
umfasst bzw. daraus besteht, wobei:
V_{H} für eine Schwere-Kette-variable-Domäne steht;
CH₁ für eine Domäne einer Schwere-Kette-konstante-Region, z. B. Domäne 1 davon, steht;
CH₂ für eine Domäne einer Schwere-Kette-konstante-Region, z. B. Domäne 2 davon, steht;
CH₃ für eine Domäne einer Schwere-Kette-konstante-Region, z. B. Domäne 3 davon, steht;
X für eine Bindung oder einen Linker steht;
Y für eine Bindung oder einen Linker steht;
V₁ für ein dsFv steht;
V_{L} für eine variable Domäne, z. B. eine Leichte-Kette-variable-Domäne steht;
C_{L} für eine Domäne aus einer konstanten Region, z. B. eine Leichte-Kette-konstante-Region-Domäne, wie z. B. Ckappa, steht;
V₂ für ein dsFv steht;
p für 0 oder 1 steht;
q für 0 oder 1 steht;
wobei p und/oder q gleich 1 ist und,
wenn p gleich 1 ist, q gleich 0 ist und Y fehlt und,
wenn q gleich 1 ist, p gleich 0 ist und X fehlt und,
wenn q gleich 1 ist und V₂ ein dsFv ist, die V_{L}-Domäne von V₂ an Y gebunden ist und
das Molekül als Dimer mit zwei schweren Ketten der Formel (I) und zwei assoziierten leichten Ketten der Formel (II) bereitgestellt ist.

2. Multispezifisches Antikörpermolekül nach Anspruch 1, wobei p gleich 1 ist.

3. Multispezifisches Antikörpermolekül nach Anspruch 1, wobei q gleich 1 ist.

4. Multispezifisches Antikörpermolekül nach Anspruch 1 oder 2, wobei X für einen Peptidlinker steht.

5. Multispezifisches Antikörpermolekül nach Anspruch 4, wobei X die Sequenz unter SEQ ID NO: 1 oder SEQ ID NO: 2 aufweist.

6. Multispezifisches Antikörpermolekül nach Anspruch 1 oder 2, wobei X für eine Bindung steht.

7. Multispezifisches Antikörpermolekül nach Anspruch 1 oder 3, wobei Y für einen Peptidlinker steht.

8. Multispezifisches Antikörpermolekül nach Anspruch 7, wobei Y die Sequenz unter SEQ ID NO: 1 oder SEQ ID NO: 2 aufweist.

9. Multispezifisches Antikörpermolekül nach Anspruch 1 oder 3, wobei Y für eine Bindung steht.

10. Multispezifisches Antikörpermolekül nach einem der Ansprüche 1 bis 9, wobei eine Intravariable-Region-Disulfidbindung in V₁ oder V₂ unabhängig ausgewählt ist aus der Gruppe umfassend oder bestehend aus: V_{H}37 und V_{L}95, V_{H}44 und V_{L}100, V_{H}44 und V_{L}105, V_{H}45 und V_{L}87, V_{H}100 and V_{L}50, V_{H}100b und V_{L}49, V_{H}98 und V_{L}46, V_{H}101 und V_{L}46, V_{H}105 und V_{L}43 und V_{H}106 und V_{L}57, wobei die V_{H}- und V_{L}-Werte unabhängig innerhalb einer gegebenen V₁ oder V₂ sind, z. B. V_{H}44 und V_{L}100, und wobei die Nummerierung nach Kabat gilt.

11. Multispezifisches Antikörpermolekül nach Anspruch 10, wobei sich die Intravariable-Region-Disulfid¬bindung in V₁ oder V₂ an Position V_{H}44 und V_{L}100 befindet.

12. Polynukleotid, codierend ein multispezifisches Antikörpermolekül nach einem der Ansprüche 1 bis 11.

13. Vektor, umfassend ein Polynukleotid mit der in Anspruch 12 angegebenen Bedeutung.

14. Wirtszelle, umfassend das Polynukleotid bzw. den Vektor gemäß Anspruch 12 bzw. 13.

15. Wirtszelle, umfassend drei Vektoren, wobei jeder Vektor ein eine unterschiedliche Polypeptidkette eines multispezifischen Antikörpermoleküls nach einem der Ansprüche 1 bis 11 codierendes Polynukleotid umfasst.

16. Verfahren, umfassend Exprimieren eines multispezifischen Antikörpermoleküls von einer Wirtszelle mit der in Anspruch 14 oder 15 angegebenen Bedeutung.

17. Pharmazeutische Zusammensetzung, umfassend ein multispezifisches Antikörpermolekül nach einem der Ansprüche 1 bis 11 und wenigstens einen Exzipienten.

18. Multispezifisches Antikörpermolekül nach einem der Ansprüche 1 bis 11 oder pharmazeutische Zusammensetzung nach Anspruch 17 zur Verwendung bei einer Behandlung.

## Revendications

1. Molécule d'anticorps multispécifique comprenant ou constituée de :
a) une chaîne polypeptidique de formule (I) :
V_{H}-CH₁-CH₂-CH₃-X-(V₁)_{P};
et
b) une chaîne polypeptidique de formule (II) :
V_{L}-C_{L}-Y-(V₂)_{q} ;
dans laquelle :
V_{H} représente un domaine variable de chaîne lourde ;
CH₁ représente un domaine d'une région constante de chaîne lourde, par exemple le domaine 1 de celle-ci ;
CH₂ représente un domaine d'une région constante de chaîne lourde, par exemple le domaine 2 de celle-ci ;
CH₃ représente un domaine d'une région constante de chaîne lourde, par exemple le domaine 3 de celle-ci ;
X représente une liaison ou un lieur ;
Y représente une liaison ou un lieur ;
V₁ représente un dsFv ;
V_{L} représente un domaine variable, par exemple un domaine variable de chaîne légère ;
C_{L} représente un domaine d'une région constante, par example un domaine de région constante de chaîne légère,
tel que Ckappa ;
V₂ représente un dsFv ;
p représente 0 ou 1 ;
q représente 0 ou 1 ;
dans laquelle au moins un parmi p ou q est 1, et
lorsque p est 1, q est 0, et y est absent, et
lorsque q est 1, p est 0, et X est absent, et
lorsque q est 1 et V₂ est un dsFv, le domaine VL de V₂ est lié à Y, et
la molécule est mise à disposition en tant que dimère présentant deux chaînes lourdes de formule (I) et deux chaînes légères associées de formule (II).

2. Molécule d'anticorps multispécifique selon la revendication 1, dans laquelle p est 1.

3. Molécule d'anticorps multispécifique selon la revendication 1, dans laquelle q est 1.

4. Molécule d'anticorps multispécifique selon la revendication 1 ou 2, dans laquelle X est un lieur peptidique.

5. Molécule d'anticorps multispécifique selon la revendication 4, dans laquelle X a la séquence de SEQ ID NO: 1 ou SEQ ID NO: 2.

6. Molécule d'anticorps multispécifique selon la revendication 1 ou 2, dans laquelle X est une liaison.

7. Molécule d'anticorps multispécifique selon la revendication 1 ou 3, dans laquelle Y est un lieur peptidique.

8. Molécule d'anticorps multispécifique selon la revendication 7, dans laquelle Y a la séquence de SEQ ID NO: 1 ou SEQ ID NO: 2.

9. Molécule d'anticorps multispécifique selon revendication 1 ou 3, dans laquelle Y est une liaison.

10. Molécule d'anticorps multispécifique selon l'une quelconque des revendications 1 à 9, dans laquelle une liaison disulfure d'une région intra-variable dans V₁ ou V₂ est indépendamment choisie dans le groupe comprenant ou constitué par : V_{H}37 et V_{L}95, V_{H}44 et V_{L}100, V_{H}44 et V_{L}105, V_{H}45 et V_{L}87, V_{H}100 et V_{L}50, V_{H}100b et V_{L}49, V_{H}98 et V_{L}46, V_{H}101 et V_{L}46, V_{H}105 et V_{L}43 et V_{H}106 et V_{L}57 les valeurs V_{H} et V_{L} étant indépendamment dans un V₁ ou un V₂ donné, par exemple V_{H}44 et V_{L}100, et la numérotation étant selon Kabat.

11. Molécule d'anticorps multispécifique selon la revendication 10, dans laquelle la liaison disulfure d'une région intra-variable dans V₁ ou V₂ se trouve en position V_{H}44 et V_{L}100.

12. Polynucléotide codant pour une molécule d'anticorps multispécifique selon l'une quelconque des revendications 1 à 11.

13. Vecteur comprenant un polynucléotide défini dans la revendication 12.

14. Cellule hôte comprenant le polynucléotide ou le vecteur selon la revendication 12 ou 13, respectivement.

15. Cellule hôte comprenant trois vecteurs, chaque vecteur comprenant un polynucléotide codant pour une chaîne polypeptidique différente d'une molécule d'anticorps multispécifique selon l'une quelconque des revendications 1 à 11.

16. Procédé comprenant l'expression d'une molécule d'anticorps multispécifique à partir d'une cellule hôte définie dans la revendication 14 ou 15.

17. Composition pharmaceutique comprenant une molécule d'anticorps multispécifique selon l'une quelconque des revendications 1 à 11 et au moins un excipient.

18. Molécule d'anticorps multispécifique selon l'une quelconque des revendications 1 à 11 ou composition pharmaceutique selon la revendication 17, pour utilisation dans un traitement.


### SEQUENCE LISTING

<110> UCB Biopharma SPRL
<120> Multispecific antibody constructs
<130> G0229_WO
<160> 70
<170> PatentIn version 3.5
<210> 1
   <211> 11
   <212> PRT
   <213> Artificial Sequence
<220>
   <223> Peptide Linker
<400> 1
   Ser Gly Gly Gly Gly Ser Gly Gly Gly Gly Ser
   1               5                   10
<210> 2
   <211> 11
   <212> PRT
   <213> Artificial Sequence
<220>
   <223> Peptide Linker
<400> 2
   Ser Gly Gly Gly Gly Thr Gly Gly Gly Gly Ser
   1               5                   10
<210> 3
   <211> 8
   <212> PRT
   <213> Artificial Sequence
<220>
   <223> Hinge linker
<400> 3
   Asp Lys Thr His Thr Cys Ala Ala
   1               5
<210> 4
   <211> 11
   <212> PRT
   <213> Artificial Sequence
<220>
   <223> Hinge linker
<400> 4
   Asp Lys Thr His Thr Cys Pro Pro Cys Pro Ala
   1               5                   10
<210> 5
   <211> 18
   <212> PRT
   <213> Artificial Sequence
<220>
   <223> Hinge linker
<400> 5
   Asp Lys Thr His Thr Cys Pro Pro Cys Pro Ala Thr Cys Pro Pro Cys
   1               5                   10                  15
   Pro Ala
<210> 6
   <211> 25
   <212> PRT
   <213> Artificial Sequence
<220>
   <223> Hinge linker
<400> 6
   Asp Lys Thr His Thr Cys Pro Pro Cys Pro Ala Thr Cys Pro Pro Cys
   1               5                   10                  15
   Pro Ala Thr Cys Pro Pro Cys Pro Ala
               20                  25
<210> 7
   <211> 30
   <212> PRT
   <213> Artificial Sequence
<220>
   <223> Hinge linker
<400> 7
   Asp Lys Thr His Thr Cys Pro Pro Cys Pro Ala Gly Lys Pro Thr Leu
   1               5                   10                  15
   Tyr Asn Ser Leu Val Met Ser Asp Thr Ala Gly Thr Cys Tyr
               20                  25                  30
<210> 8
   <211> 31
   <212> PRT
   <213> Artificial Sequence
<220>
   <223> Hinge linker
<400> 8
   Asp Lys Thr His Thr Cys Pro Pro Cys Pro Ala Gly Lys Pro Thr His
   1 5 10 15
   Val Asn Val Ser Val Val Met Ala Glu Val Asp Gly Thr Cys Tyr
   20 25 30
<210> 9
   <211> 15
   <212> PRT
   <213> Artificial Sequence
<220>
   <223> Hinge linker
<400> 9
   Asp Lys Thr His Thr Cys Cys Val Glu Cys Pro Pro Cys Pro Ala
   1 5 10 15
<210> 10
   <211> 26
   <212> PRT
   <213> Artificial Sequence
<220>
   <223> Hinge linker
<400> 10
   Asp Lys Thr His Thr Cys Pro Arg Cys Pro Glu Pro Lys Ser Cys Asp
   1 5 10 15
   Thr Pro Pro Pro Cys Pro Arg Cys Pro Ala
   20 25
<210> 11
   <211> 11
   <212> PRT
   <213> Artificial Sequence
<220>
   <223> Hinge linker
<400> 11
   Asp Lys Thr His Thr Cys Pro Ser Cys Pro Ala
   1 5 10
<210> 12
   <211> 7
   <212> PRT
   <213> Artificial Sequence
<220>
   <223> Flexible linker
<400> 12
   Ser Gly Gly Gly Gly Ser Glu
   1 5
<210> 13
   <211> 6
   <212> PRT
   <213> Artificial Sequence
<220>
   <223> Flexible linker
<400> 13
   Asp Lys Thr His Thr Ser
   1 5
<210> 14
   <211> 6
   <212> PRT
   <213> Artificial Sequence
<220>
   <223> Flexible linker
<400> 14
   Ser Gly Gly Gly Gly Ser
   1 5
<210> 15
   <211> 11
   <212> PRT
   <213> Artificial Sequence
<220>
   <223> Flexible linker
<400> 15
   Ser Gly Gly Gly Gly Ser Gly Gly Gly Gly Ser
   1 5 10
<210> 16
   <211> 16
   <212> PRT
   <213> Artificial Sequence
<220>
   <223> Flexible linker
<400> 16
   Ser Gly Gly Gly Gly Ser Gly Gly Gly Gly Ser Gly Gly Gly Gly Ser
   1 5 10 15
<210> 17
   <211> 21
   <212> PRT
   <213> Artificial Sequence
<220>
   <223> Flexible linker
<400> 17
   Ser Gly Gly Gly Gly Ser Gly Gly Gly Gly Ser Gly Gly Gly Gly Ser
   1               5                   10                  15
   Gly Gly Gly Gly Ser
               20
<210> 18
   <211> 26
   <212> PRT
   <213> Artificial Sequence
<220>
   <223> Flexible linker
<400> 18
   Ser Gly Gly Gly Gly Ser Gly Gly Gly Gly Ser Gly Gly Gly Gly Ser
   1               5                   10                  15
   Gly Gly Gly Gly Ser Gly Gly Gly Gly Ser
               20                  25
<210> 19
   <211> 11
   <212> PRT
   <213> Artificial Sequence
<220>
   <223> Flexible linker
<400> 19
   Ala Ala Ala Gly Ser Gly Gly Ala Ser Ala Ser
   1               5                   10
<210> 20
   <211> 16
   <212> PRT
   <213> Artificial Sequence
<220>
   <223> Flexible linker
<220>
   <221> misc_feature
   <222> (7)..(7)
   <223> Xaa can be naturally occuring amino acid
<400> 20
   Ala Ala Ala Gly Ser Gly Xaa Gly Gly Gly Ser Gly Ala Ser Ala Ser
   1               5                   10                  15
<210> 21
   <211> 21
   <212> PRT
   <213> Artificial Sequence
<220>
   <223> Flexible linker
<220>
   <221> misc_feature
   <222> (7)..(7)
   <223> Xaa can be naturally occuring amino acid
<220>
   <221> misc_feature
   <222> (12)..(12)
   <223> Xaa can be naturally occuring amino acid
<400> 21
   Ala Ala Ala Gly Ser Gly Xaa Gly Gly Gly Ser Xaa Gly Gly Gly Ser
   1               5                   10                  15
   Gly Ala Ser Ala Ser
               20
<210> 22
   <211> 26
   <212> PRT
   <213> Artificial Sequence
<220>
   <223> Flexible linker
<220>
   <221> misc_feature
   <222> (7)..(7)
   <223> Xaa can be naturally occuring amino acid
<220>
   <221> misc_feature
   <222> (12)..(12)
   <223> Xaa can be naturally occuring amino acid
<220>
   <221> misc_feature
   <222> (17)..(17)
   <223> Xaa can be naturally occuring amino acid
<400> 22
   Ala Ala Ala Gly Ser Gly Xaa Gly Gly Gly Ser Xaa Gly Gly Gly Ser
   1               5                   10                  15
   Xaa Gly Gly Gly Ser Gly Ala Ser Ala Ser
               20                  25
<210> 23
   <211> 31
   <212> PRT
   <213> Artificial Sequence
<220>
   <223> Flexible linker
<220>
   <221> misc_feature
   <222> (7)..(7)
   <223> Xaa can be naturally occuring amino acid
<220>
   <221> misc_feature
   <222> (12)..(12)
   <223> Xaa can be naturally occuring amino acid
<220>
   <221> misc_feature
   <222> (17)..(17)
   <223> Xaa can be naturally occuring amino acid
<220>
   <221> misc_feature
   <222> (22)..(22)
   <223> Xaa can be naturally occuring amino acid
<400> 23
   Ala Ala Ala Gly Ser Gly Xaa Gly Gly Gly Ser Xaa Gly Gly Gly Ser
   1               5                   10                  15
   Xaa Gly Gly Gly Ser Xaa Gly Gly Gly Ser Gly Ala Ser Ala Ser
               20                  25                  30
<210> 24
   <211> 13
   <212> PRT
   <213> Artificial Sequence
<220>
   <223> Flexible linker
<220>
   <221> misc_feature
   <222> (7)..(7)
   <223> Xaa can be naturally occuring amino acid
<400> 24
   Ala Ala Ala Gly Ser Gly Xaa Ser Gly Ala Ser Ala Ser
   1 5 10
<210> 25
   <211> 28
   <212> PRT
   <213> Artificial Sequence
<220>
   <223> Flexible linker
<400> 25
   Pro Gly Gly Asn Arg Gly Thr Thr Thr Thr Arg Arg Pro Ala Thr Thr
   1 5 10 15
   Thr Gly Ser Ser Pro Gly Pro Thr Gln Ser His Tyr
   20 25
<210> 26
   <211> 11
   <212> PRT
   <213> Artificial Sequence
<220>
   <223> Flexible linker
<400> 26
   Ala Thr Thr Thr Gly Ser Ser Pro Gly Pro Thr
   1 5 10
<210> 27
   <211> 6
   <212> PRT
   <213> Artificial Sequence
<220>
   <223> Flexible linker
<400> 27
   Ala Thr Thr Thr Gly Ser
   1 5
<210> 28
   <211> 21
   <212> PRT
   <213> Artificial Sequence
<220>
   <223> Flexible linker
<400> 28
   Glu Pro Ser Gly Pro Ile Ser Thr Ile Asn Ser Pro Pro Ser Lys Glu
   1 5 10 15
   Ser His Lys Ser Pro
   20
<210> 29
   <211> 15
   <212> PRT
   <213> Artificial Sequence
<220>
   <223> Flexible linker
<400> 29
   Gly Thr Val Ala Ala Pro Ser Val Phe Ile Phe Pro Pro Ser Asp
   1 5 10 15
<210> 30
   <211> 15
   <212> PRT
   <213> Artificial Sequence
<220>
   <223> Flexible linker
<400> 30
   Gly Gly Gly Gly Ile Ala Pro Ser Met Val Gly Gly Gly Gly Ser
   1 5 10 15
<210> 31
   <211> 15
   <212> PRT
   <213> Artificial Sequence
<220>
   <223> Flexible linker
<400> 31
   Gly Gly Gly Gly Lys Val Glu Gly Ala Gly Gly Gly Gly Gly Ser
   1               5                   10                  15
<210> 32
   <211> 15
   <212> PRT
   <213> Artificial Sequence
<220>
   <223> Flexible linker
<400> 32
   Gly Gly Gly Gly Ser Met Lys Ser His Asp Gly Gly Gly Gly Ser
   1 5 10 15
<210> 33
   <211> 15
   <212> PRT
   <213> Artificial Sequence
<220>
   <223> Flexible linker
<400> 33
   Gly Gly Gly Gly Asn Leu Ile Thr Ile Val Gly Gly Gly Gly Ser
   1               5                   10                  15
<210> 34
   <211> 15
   <212> PRT
   <213> Artificial Sequence
<220>
   <223> Flexible linker
<400> 34
   Gly Gly Gly Gly Val Val Pro Ser Leu Pro Gly Gly Gly Gly Ser
   1               5                   10                  15
<210> 35
   <211> 12
   <212> PRT
   <213> Artificial Sequence
<220>
   <223> Flexible linker
<400> 35
   Gly Gly Glu Lys Ser Ile Pro Gly Gly Gly Gly Ser
   1               5                   10
<210> 36
   <211> 18
   <212> PRT
   <213> Artificial Sequence
<220>
   <223> Flexible linker
<400> 36
   Arg Pro Leu Ser Tyr Arg Pro Pro Phe Pro Phe Gly Phe Pro Ser Val
   1               5                   10                  15
   Arg Pro
<210> 37
   <211> 18
   <212> PRT
   <213> Artificial Sequence
<220>
   <223> Flexible linker
<400> 37
   Tyr Pro Arg Ser Ile Tyr Ile Arg Arg Arg His Pro Ser Pro Ser Leu
   1               5                   10                  15
   Thr Thr
<210> 38
   <211> 18
   <212> PRT
   <213> Artificial Sequence
<220>
   <223> Flexible linker
<400> 38
   Thr Pro Ser His Leu Ser His Ile Leu Pro Ser Phe Gly Leu Pro Thr
   1               5                   10                  15
   Phe Asn
<210> 39
   <211> 18
   <212> PRT
   <213> Artificial Sequence
<220>
   <223> Flexible linker
<400> 39
   Arg Pro Val Ser Pro Phe Thr Phe Pro Arg Leu Ser Asn Ser Trp Leu
   1               5                   10                  15
   Pro Ala
<210> 40
   <211> 18
   <212> PRT
   <213> Artificial Sequence
<220>
   <223> Flexible linker
<400> 40
   Ser Pro Ala Ala His Phe Pro Arg Ser Ile Pro Arg Pro Gly Pro Ile
   1 5 10 15
   Arg Thr
<210> 41
   <211> 18
   <212> PRT
   <213> Artificial Sequence
<220>
   <223> Flexible linker
<400> 41
   Ala Pro Gly Pro Ser Ala Pro Ser His Arg Ser Leu Pro Ser Arg Ala
   1 5 10 15
   Phe Gly
<210> 42
   <211> 18
   <212> PRT
   <213> Artificial Sequence
<220>
   <223> Flexible linker
<400> 42
   Pro Arg Asn Ser Ile His Phe Leu His Pro Leu Leu Val Ala Pro Leu
   1 5 10 15
   Gly Ala
<210> 43
   <211> 18
   <212> PRT
   <213> Artificial Sequence
<220>
   <223> Flexible linker
<400> 43
   Met Pro Ser Leu Ser Gly Val Leu Gln Val Arg Tyr Leu Ser Pro Pro
   1 5 10 15
   Asp Leu
<210> 44
   <211> 18
   <212> PRT
   <213> Artificial Sequence
<220>
   <223> Flexible linker
<400> 44
   Ser Pro Gln Tyr Pro Ser Pro Leu Thr Leu Thr Leu Pro Pro His Pro
   1 5 10 15
   Ser Leu
<210> 45
   <211> 18
   <212> PRT
   <213> Artificial Sequence
<220>
   <223> Flexible linker
<400> 45
   Asn Pro Ser Leu Asn Pro Pro Ser Tyr Leu His Arg Ala Pro Ser Arg
   1 5 10 15
   Ile Ser
<210> 46
   <211> 17
   <212> PRT
   <213> Artificial Sequence
<220>
   <223> Flexible linker
<400> 46
   Leu Pro Trp Arg Thr Ser Leu Leu Pro Ser Leu Pro Leu Arg Arg Arg
   1 5 10 15
   Pro
<210> 47
   <211> 18
   <212> PRT
   <213> Artificial Sequence
<220>
   <223> Flexible linker
<400> 47
   Pro Pro Leu Phe Ala Lys Gly Pro Val Gly Leu Leu Ser Arg Ser Phe
   1 5 10 15
   Pro Pro
<210> 48
   <211> 18
   <212> PRT
   <213> Artificial Sequence
<220>
   <223> Flexible linker
<400> 48
   Val Pro Pro Ala Pro Val Val Ser Leu Arg Ser Ala His Ala Arg Pro
   1 5 10 15
   Pro Tyr
<210> 49
   <211> 17
   <212> PRT
   <213> Artificial Sequence
<220>
   <223> Flexible linker
<400> 49
   Leu Arg Pro Thr Pro Pro Arg Val Arg Ser Tyr Thr Cys Cys Pro Thr
   1 5 10 15
   Pro
<210> 50
   <211> 18
   <212> PRT
   <213> Artificial Sequence
<220>
   <223> Flexible linker
<400> 50
   Pro Asn Val Ala His Val Leu Pro Leu Leu Thr Val Pro Trp Asp Asn
   1 5 10 15
   Leu Arg
<210> 51
   <211> 18
   <212> PRT
   <213> Artificial Sequence
<220>
   <223> Flexible linker
<400> 51
   Cys Asn Pro Leu Leu Pro Leu Cys Ala Arg Ser Pro Ala Val Arg Thr
   1 5 10 15
   Phe Pro
<210> 52
   <211> 11
   <212> PRT
   <213> Artificial Sequence
<220>
   <223> rigid linker
<400> 52
   Gly Ala Pro Ala Pro Ala Ala Pro Ala Pro Ala
   1 5 10
<210> 53
   <211> 4
   <212> PRT
   <213> Artificial Sequence
<220>
   <223> rigid linker
<400> 53
   Pro Pro Pro Pro
   1
<210> 54
   <211> 11
   <212> PRT
   <213> Artificial Sequence
<220>
   <223> albumin binding peptide
<400> 54
   Asp Leu Cys Leu Arg Asp Trp Gly Cys Leu Trp
   1 5 10
<210> 55
   <211> 11
   <212> PRT
   <213> Artificial Sequence
<220>
   <223> albumin binding peptide
<400> 55
   Asp Ile Cys Leu Pro Arg Trp Gly Cys Leu Trp
   1 5 10
<210> 56
   <211> 15
   <212> PRT
   <213> Artificial Sequence
<220>
   <223> albumin binding peptide
<400> 56
   Met Glu Asp Ile Cys Leu Pro Arg Trp Gly Cys Leu Trp Gly Asp
   1 5 10 15
<210> 57
   <211> 20
   <212> PRT
   <213> Artificial Sequence
<220>
   <223> albumin binding peptide
<400> 57
   Gln Arg Leu Met Glu Asp Ile Cys Leu Pro Arg Trp Gly Cys Leu Trp
   1 5 10 15
   Glu Asp Asp Glu
   20
<210> 58
   <211> 20
   <212> PRT
   <213> Artificial Sequence
<220>
   <223> albumin binding peptide
<400> 58
   Gln Gly Leu Ile Gly Asp Ile Cys Leu Pro Arg Trp Gly Cys Leu Trp
   1 5 10 15
   Gly Arg Ser Val
   20
<210> 59
   <211> 21
   <212> PRT
   <213> Artificial Sequence
<220>
   <223> albumin binding peptide
<400> 59
   Gln Gly Leu Ile Gly Asp Ile Cys Leu Pro Arg Trp Gly Cys Leu Trp
   1 5 10 15
   Gly Arg Ser Val Lys
   20
<210> 60
   <211> 15
   <212> PRT
   <213> Artificial Sequence
<220>
   <223> albumin binding peptide
<400> 60
   Glu Asp Ile Cys Leu Pro Arg Trp Gly Cys Leu Trp Glu Asp Asp
   1 5 10 15
<210> 61
   <211> 18
   <212> PRT
   <213> Artificial Sequence
<220>
   <223> albumin binding peptide
<400> 61
   Arg Leu Met Glu Asp Ile Cys Leu Pro Arg Trp Gly Cys Leu Trp Glu
   1 5 10 15
   Asp Asp
<210> 62
   <211> 16
   <212> PRT
   <213> Artificial Sequence
<220>
   <223> albumin binding peptide
<400> 62
   Met Glu Asp Ile Cys Leu Pro Arg Trp Gly Cys Leu Trp Glu Asp Asp
   1 5 10 15
<210> 63
   <211> 15
   <212> PRT
   <213> Artificial Sequence
<220>
   <223> albumin binding peptide
<400> 63
   Met Glu Asp Ile Cys Leu Pro Arg Trp Gly Cys Leu Trp Glu Asp
   1               5                   10                  15
<210> 64
   <211> 18
   <212> PRT
   <213> Artificial Sequence
<220>
   <223> albumin binding peptide
<400> 64
   Arg Leu Met Glu Asp Ile Cys Leu Ala Arg Trp Gly Cys Leu Trp Glu
   1               5                   10                  15
   Asp Asp
<210> 65
   <211> 20
   <212> PRT
   <213> Artificial Sequence
<220>
   <223> albumin binding peptide
<400> 65
   Glu Val Arg Ser Phe Cys Thr Arg Trp Pro Ala Glu Lys Ser Cys Lys
   1               5                   10                  15
   Pro Leu Arg Gly
               20
<210> 66
   <211> 20
   <212> PRT
   <213> Artificial Sequence
<220>
   <223> albumin binding peptide
<400> 66
   Arg Ala Pro Glu Ser Phe Val Cys Tyr Trp Glu Thr Ile Cys Phe Glu
   1               5                   10                  15
   Arg Ser Glu Gln
               20
<210> 67
   <211> 11
   <212> PRT
   <213> Artificial Sequence
<220>
   <223> albumin binding peptide
<400> 67
   Glu Met Cys Tyr Phe Pro Gly Ile Cys Trp Met
   1               5                   10
<210> 68
   <211> 20
   <212> PRT
   <213> Artificial Sequence
<220>
   <223> albumin binding peptide
<400> 68
   Gly Gly Gly Gly Ser Gly Gly Gly Gly Ser Gly Gly Gly Gly Ser Gly
   1               5                   10                  15
   Gly Gly Gly Ser
               20
<210> 69
   <211> 16
   <212> PRT
   <213> Artificial Sequence
<220>
   <223> flexible linker
<400> 69
   Ser Gly Gly Gly Gly Ser Gly Gly Gly Gly Ser Gly Gly Gly Gly Ser
   1               5                   10                  15
<210> 70
   <211> 16
   <212> PRT
   <213> Artificial Sequence
<220>
   <223> flexible linker
<400> 70
   Ser Gly Gly Gly Gly Ser Gly Gly Gly Gly Thr Gly Gly Gly Gly Ser
   1               5                   10                  15